(19) Europäisches Patentamt
European Patent Office
Office européen des brevets

(11) Veröffentlichungsnummer : **0 411 736 B1**

(12) # EUROPÄISCHE PATENTSCHRIFT

(45) Veröffentlichungstag der Patentschrift : **04.05.94 Patentblatt 94/18**

(51) Int. Cl.$^5$ : **C07J 53/00,** A61K 31/565, A61K 31/58

(21) Anmeldenummer : **90250202.0**

(22) Anmeldetag : **06.08.90**

(54) **11 Beta-substituierte 16 alpha, 17alpha-Methylen-estra-4,9-dien-3-one.**

(30) Priorität : **04.08.89 DD 331481**
**16.08.89 DD 331819**

(43) Veröffentlichungstag der Anmeldung : **06.02.91 Patentblatt 91/06**

(45) Bekanntmachung des Hinweises auf die Patenterteilung : **04.05.94 Patentblatt 94/18**

(84) Benannte Vertragsstaaten : **AT BE CH DE DK ES FR GB GR IT LI LU NL SE**

(56) Entgegenhaltungen :
**US-A- 3 646 074**
**THE JOURNAL OF ORGANIC CHEMISTRY, Band 36, Nr. 14, Juli 1971, Seiten 1952-1962; W.F. JOHNS et al.: "Synthesis and reactions of 17beta-oxygenated 16alpha,17-cyclopropylandrostanes"**

(73) Patentinhaber : **SCHERING AKTIENGESELLSCHAFT**
**D-13342 Berlin (DE)**

(72) Erfinder : **Kasch, Helmut, Dr.**
**Hermann-Duncker-Strasse 14**
**DD-6902 Jena (DD)**
Erfinder : **Krieg, Reimar, Dr.**
**A.-Reichwein-Strasse 34**
**DD-6900 Jena (DD)**
Erfinder : **Kurischko, Anatoli**
**Westendstrasse 2a**
**DD-6900 Jena (DD)**
Erfinder : **Ponsold, Kurt, Prof. Dr.,**
**Thomas-Mann-Strasse 13a**
**DD-6900 Jena (DD)**

**EP 0 411 736 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft 11β-substituierte 16α,17α-Methylen-estra-4,9-dien-3-one der allgemeinen Formel I

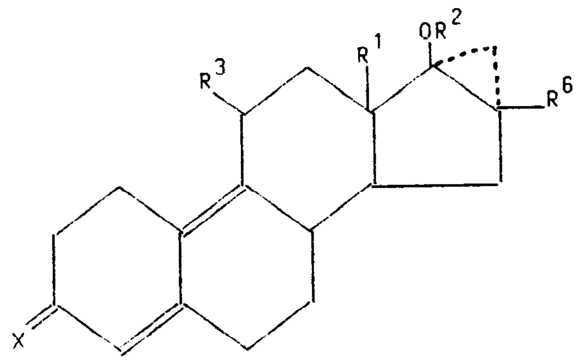

(I)

worin

$R^1$ eine Methyl- oder Ethylgruppe,

$R^2$ ein Wasserstoff, eine Alkyl-, Alkoxymethyl-, Alkanoyl-, Alkoxycarbonylgruppe - jeweils mit einer Kohlenstoffkette von 1 bis 6 Kohlenstoffatomen, 2-Methoxyethyl-, 2-Hydroxyethyl-, 2-Alkanoyloxyethyl-, (Alkanoyl = $C_1$-$C_4$) oder eine Trialkylsilylgruppe mit Alkylresten von 1 bis 4 Kohlenstoffatomen,

$R^3$ eine Vinyl-, $C_1$- bis $C_6$-Alkyl-Gruppe oder einen para-substituierten Phenylrest mit $-OCH_3$, $-SCH_3$, $-N(CH_3)_2$, $-NHCH_3$, -CN, -CHO, $CH_3CO$, $CH_3CHOH$ oder $CH_2OH$ als para-Substituent,

$R^6$ ein Wasserstoffatom oder eine Alkylgruppe mit 1 bis 4 Kohlenstoffatomen sowie

X ein Sauerstoffatom, eine Hydroxy- oder Methoxyiminogruppierung (N-OH bzw. $N-OCH_3$) oder ein cyclisches Thioketal mit 2 oder 3 Kohlenstoffringatomen bedeutet.

Aus der US-A-3 646 074 ist bekannt, daß 17β-Ethoxy-16α,17α-methylen-estra-4,9-dien-3-on androgene und anabole Eigenschaften aufweist. Die zuletzt genannte Verbindung ist auch in J. Org. Chem., Band 36, Nr. 14, **1971**, Seiten 1952-1962 beschrieben.

Vorzugsweise bedeutet in Formel (I)

$R^1$ eine Methylgruppe,

$R^2$ ein Wasserstoffatom, eine $C_1$- bis $C_6$-Alkylgruppe, eine Trialkylsilylgruppe mit Alkylresten mit 1 bis 4 Kohlenstoffatomen, eine Acetyl-, $-CH_2OCH_3$- oder 2-Methoxyethylgruppe,

$R^3$ ein Vinyl- oder para-substituierter Phenylrest mit $-N(CH_3)_2$, -CHO, $-C(O)CH_3$, $-OCH_3$ als para-Substituent,

$R^6$ ein Wasserstoffatom oder eine Methylgruppe sowie

X ein Sauerstoffatom.

Insbesondere bevorzugt sind die erfindungsgemäßen Verbindungen

- 17β-Ethoxy-11β-(4-methoxyphenyl)-16α,17α-methylen-estra-4,9-dien-3-on,
- 11β-(4-Dimethylaminophenyl)-17β-methoxy-16α,17α-methylen-estra-4,9-dien-3-on,
- 11β-(4-Dimethylaminophenyl)-17β-ethoxy-16α,17α-methylen-estra-4,9-dien-3-on,
  17β-Butyloxy-11β-(4-dimethylaminopohenyl)-16α,17α-methylen-estra-4,9-dien-3-on,
- 11β-(4-Acetylphenyl)-17β-methoxy-16α,17α-methylen-estra-4,9-dien-3-on,
- 11β-(4-Acetylphenyl)-17β-ethoxy-16α,17α-methylen-estra-4,9-dien-3-on,
- 11β-(4-Acetylphenyl)-17β-butyloxy-16α,17α-methylen-estra-4,9-dien-3-on,
- 11β-(4-Acetylphenyl)-17β-hexyloxy-16α,17α-methylen-estra-4,9-dien-3-on,
- 11β-(4-Acetylphenyl)-17β-methoxyethyloxy-16α,17α-methylen-estra-4,9-dien-3-on,
- 11β-(4-Formylphenyl)-17β-methoxy-16α,17α-methylen-estra-4,9-dien-3-on sowie
- 17β-Ethoxy-11β-(4-formylphenyl)-16α,17α-methylen-estra-4,9-dien-3-on,
- 17β-(Dimethyl-tert.butyl-siloxy)-11β-(4-methoxyphenyl)-16α,17α-methylen-estra-4,9-dien-3-on,
- 11β-(4-Dimethylaminophenyl)-17β-(dimethyl-tert.butyl-siloxy)-16α,17α-methylen-estra-4,9-dien-3-on,
- 11β-(4-Acetylphenyl)-17β-(dimethyl-tert.butyl-siloxy)-16α,17α-methylen-estra-4,9-dien-3-on,
- 17β-Hydroxy-11β-(4-methoxyphenyl)-16α,17α-methylen-estra-4,9-dien-3-on,
- 11β-(4-Dimethylaminophenyl)-17β-hydroxy-16α,17α-methylen-estra-4,9-dien-3-on,
- 11β-(4-Acetylphenyl)-17β-hydroxy-16α,17α-methylen-estra-4,9-dien-3-on,
- 17β-Acetoxy-11β-(4-dimethylaminophenyl)-16α,17α-methylen-estra-4,9-dien-3-on,

- 17β-Acetoxy-11β-(4-acetylphenyl)-16α,17α-methylen-estra-4,9-dien-3-on,
- 11β-(4-Dimethylaminophenyl)-17β-methoxymethyl-16α,17α-methylen-estra-4,9-dien-3-on,
- 11β-(4-Acetylphenyl)-17β-methoxymethyl-16α,17α-methylen-estra-4,9-dien-3-on,
- 11β-(4-Dimethylaminophenyl)-17β-methoxy-16β-methyl-16α,17α-methylen-estra-4,9-dien-3-on sowie
- 11β-(4-Acetylphenyl)-17β-methoxy-16α,17α-methylen-estra-4,9-dien-3-on.

Die Verbindungen der allgemeinen Formel I besitzen eine starke Affinität zum Gestagenrezeptor, ohne selbst gestagene Aktivität zu entfalten. Sie sind kompetitive Antagonisten des Progesterons (Antigestagene); da sie das zur Aufrechterhaltung der Schwangerschaft erforderliche Progesteron vom Rezeptor verdrängen, sind sie zur Auslösung von Aborten und zur Einleitung der Geburt geeignet.

Neben den genannten Indikationen können die erfindungsgemäßen Verbindungen auch zur Behandlung der Endometriose, Dysmenorrhoe und endokriner hormonabhnängiger Tumore, wie z.B. Mamma-Carcinom und Meningeom verwendet werden.

Zur Kennzeichnung der antigestagenen Wirkung diente die tierexperimentell ermittelte abortive Wirkung der Substanzen. Zu diesem Zweck wurden weibliche gravide Ratten (positiver Spermiennachweis =1 Graviditätstag) im Gewicht zwischen 180 und 200 g am 5. bis 8. Graviditätstag mit der Testverbindung in Erdnußöl suspendiert subkutan behaldelt. Nach Autopsie am 20. Graviditätstag wurden die Uterie untersucht. Dabei wurde die Anzahl der graviden Weibchen und die durchschnittliche Zahl der Feten pro gravides Tier festgestellt. Der Hemmeffekt wurde wie folgt berechnet:

$$He = \left(1 - \frac{x_v \cdot n_k}{m_v \cdot x_k}\right) \cdot 100\ (\%)$$

x = Anzahl der graviden Weibchen
n = Anzahl der besamten Weibchen
v = Versuchsgruppe
k = Kontrollgruppe

| Gruppe Substanz | Gesamtdosis (mg/Tier/4d) | N | Fertilitätshemmung absol. | rel. % |
|---|---|---|---|---|
| 17β-Methoxy-11β-(acetylphenyl) | 3 | 6 | 6 | 100 |
| 17β-Methoxy-11β-(dimethylaminophenyl) | 3 | 6 | 6 | 100 |
| 17β-Ethoxy-11β-(acetylphenyl) | 3 | 6 | 6 | 100 |
| 17β-Ethoxy-11β-(dimethylaminophenyl) | 3 | 6 | 4 | 67 |
| 17β-(methoxy-etyhl-11β-(acetylphenyl) | 3 | 6 | 6 | 100 |
| Kontrolle | --- | 6 | 0 | 0 |
| Vergleich (RU 486*) | 3 | 6 | 6 | 100 |

*) 11ß-(4-Dimethylaminophenyl)-17ß-hydroxy-17α-(prop-1-inyl)-4,9-estradien-3-on; EP-A-0 057 115

Die erfindungsgemäßen Verbindungen der allgemeinen Formel I können in Form pharmazeutischer Präparate Verwendung finden. Die Herstellung der Präparate erfolgt nach an sich bekannten Methoden der Galenik durch Mischen mit organischem oder anorganischem inertem Trägermaterial, welches für die enterale, perkutane oder parenterale Applikation geeignet ist.

Die Dosierung der erfindungsgemäßen Verbindungen für die angegebenen Indikationen liegt zwischen 1 und 1000 mg täglich.

Die Verbindungen der allgemeinen Formel I werden erfindungsgemäß hergestellt, indem eine Verbindung der allgemeinen Formel XI

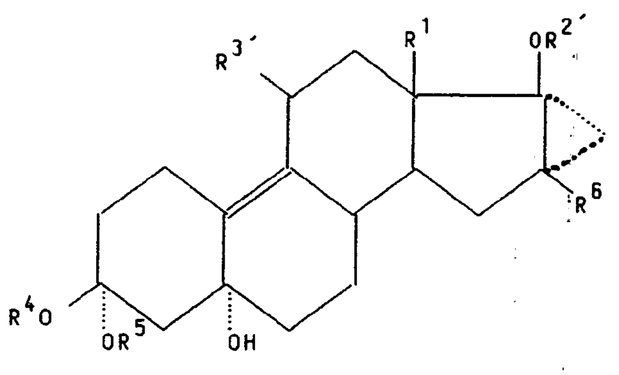

(XI),

worin

$R^1$ ein Wasserstoffatom oder eine Methylgruppe und

$R^4$ und $R^5$ je eine Methyl- oder Ethylgruppe oder gemeinsam eine Ethylen- oder 2,2-Dialkylpropylengruppe, insbesondere 2,2-Dimethylpropylengruppe,bedeuten, sowie

$R^{2'}$ und $R^{3'}$ dieselbe Bedeutung wie $R^2$ und $R^3$ in Formel I haben, wobei gegebenenfalls vorhandene Ketogruppen geschützt sind,

letztere durch Säurebehandlung in einem mit Wasser mischbaren Lösungsmittel in 11β-substituierte 16α,17α-Methylen-estra-4,9-dien-3-one der allgemeinen Formel I überführt sowie diese anschließend gegebenenfalls durch Oximierung, Thioketalisierung oder Acylierung zu einer anderen Verbindung der allgemeinen Formel I derivatisiert wird.

Als Säuren für die Säurebehandlung werden z.B. wäßrige Essigsäure, p-Toluolsulfonsäure oder Mineralsäuren wie Salzsäure, Schwefelsäure, Phosphorsäure oder Perchlorsäure und als Lösungsmittel wäßriges Methanol, Ethanol oder Aceton, verwendet. Gegebenenfalls wird während der Säurebehandlung auf 60° C bis 70° C erwärmt.

Die weitere Derivatisierung erfolgt durch Oximierung oder Thioketalisierung in 3-Stellung und bei Vorhandensein einer 17β-Hydroxygruppe gegebenenfalls durch deren Acylierung, wobei weitere erfindungsgemäße Verbindungen der allgemeinen Formel I gebildet werden.

Gegebenenfalls werden die dafür benötigten 17β-Hydroxyverbindungen durch Verseifung der entsprechenden Silylalkylether mit Pyridiniumtosylat in Methanol hergestellt.

Die Thioketalisierung wird mit Ethandithiol oder Propandithiol in Gegenwart einer Lewis-Säure wie Bortrifluoridetherat vorgenommen.

Die Oximierung erfolgt mit Hydroxylaminhydrochlorid oder Methoxyaminhydrochlorid in alkoholischer Lösung in Gegenwart einer Base wie $Na_2CO_3$, $K_2CO_3$, verdünnter NaOH oder KOH.

Die Acylierung der 17β-OH-Gruppe erfolgt mit den entsprechenden Säureanhydrid oder -chlorid in Gegenwart katalytischer Mengen einer Pyridinbase, wie Pyridin selbst oder 4-(Dimethylamino)-pyridin.

Zur Herstellung der erfindungsgemäß zu verwendenden Ausgangsprodukte der allgemeinen Formel XI erfolgt gemäß nachstehendem Schema:

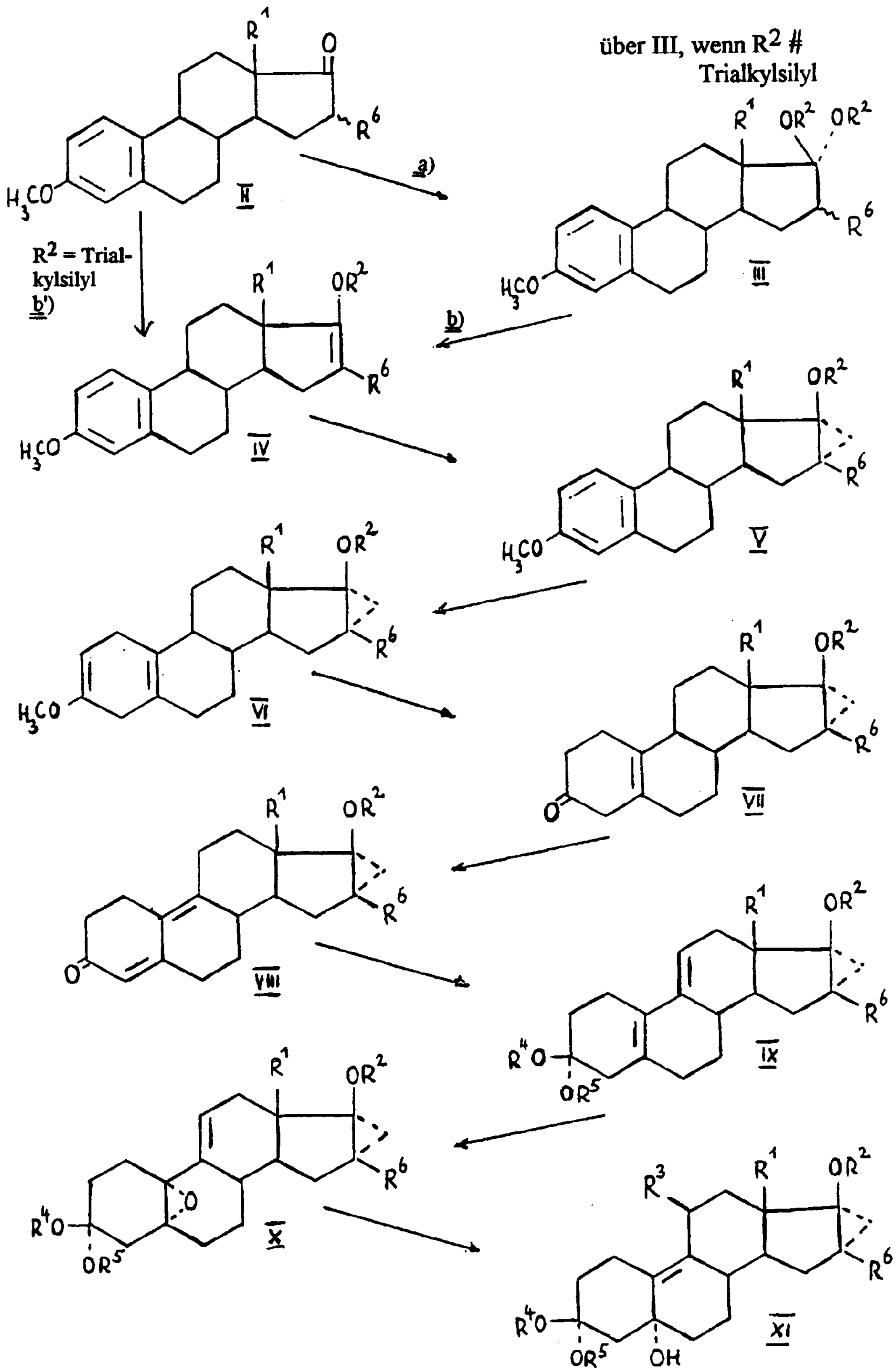


Es wird je nach gewünschten 17-Substituenten entweder

a) 3-Methoxy-gona-1,3,5(10)-trien-17-one der allgemeinen Formel II mittels eines Alkohols in Gegenwart katalytischer Mengen einer Säure in die 17-Ketale der allgemeinen Formel III überführt,

b) diese Ketale durch Thermolyse bei 120° C bis 180° C zu den 17-Alkylenolethern der allgemeinen Formel IV umgesetzt, oder

b')3-Methoxy-gona-1,3,5(10)-trien-17-one der allgemeinen Formel II mit Trialkylsilylhalogeniden oder -trifluormethansulfonaten in Gegenwart einer Base direkt in die Silylenolether der allgemeinen Formel IV überführt, worin $R_2$ für Trialkylsilyl steht,

c) die 17-Enolether IV nach Simmons-Smith in die 16α,17α-Methylenverbindungen der allgemeinen Formel V umgewandelt,

(d) die 16α,17α-Methylenverbindungen V durch Birch-Reduktion zu den 3-Enolethern der allgemeinen Formel VI reduziert,

e) aus den 3-Enolethern VI mittels katalytischer Säuremengen in einem wäßrig organischen Lösungsmittel die 3-Keto-5(10)-verbindungen der allgemeinen Formel VII hergestellt,

f) die 3-Keto-5(10)-verbindungen VII durch Bromierung/Dehydrobromierung zu den 16α,17α-Methylen-estra-4,9-dien-3-onen der allgemeinen Formel VIII umgesetzt,

g) die 16α,17α-Methylen-estra-4,9-dien-3-one VIII durch Ketalisierung mit einem Alkohol in Gegenwart katalytischer Mengen einer Säure in die Ketale der allgemeinen Formel IX, worin $R_4 = R_5 = CH_3$, $C_2H_5$ oder ein cyclisches Ketal mit 2 oder 3 C-Ringatomen bedeutet, überführt,

h) aus diesen Ketalen IX durch Epoxidierung die 5α,10α-Epoxide der allgemeinen Formel X hergestellt und

i) die 5α,10α-Epoxide X mit Arylmagnesiumhalogenid in Gegenwart eines $Cu^I$-Salzes bei einer Reaktionstemperatur von -30° C bis +30° C zu den 11β-Aryl-16α,17α-methylenverbindungen der allgemeinen Formel XI aryliert.

Bevorzugt werden im Verfahrensschritt a)
als Alkohol Methanol, Ethanol, Propanol, Butanol, Pentanol, Hexanol, Ethylenglykolmonomethylether und Essigsäuremonoglykolester, als Säuren Schwefelsäure und p-Toluensulfonsäure verwendet und die Reaktion in Gegenwart wasserentziehender Mittel, wie Ameisensäuretrimethyl- und Ameisensäuretriethylester, sowie gegebenenfalls unter Zusatz eines Lösungsmittels, wie Methylenchlorid, Chloroform, Benzol, Toluol, Xylol oder Mesitylen, durchgeführt. Im Verfahrensschritt b) wird die Thermolyse gegebenenfalls unter Zusatz von Säure, wie p-Toluolsulfonsäure, Schwefelsäure oder $KHSO_4$, sowie eines hochsiedenden Schleppmittels, wie Xylol oder Mesitylen, durchgeführt, dabei wird das Lösungsmittel gegebenenfalls unter vermindertem Druck kontinuierlich entfernt.

Wird für die ersten beiden Reaktionsschritte die Variante b') gewählt, d.h. wenn in der Verbindung der allgemeinen Formel IV $R^2$ ein Trialkylsilylrest sein soll, setzt man vorzugsweise als Trialkylsilylhalogenid Trimethylsilyl- oder Dimethyl-tert.-butylsilyl in Form der Chloride, Bromide oder Iodide, wobei die reaktionsfreudigen Trialkylsilyliodide in situ aus den Trialkylsilylchloriden und NaI hergestellt werden, als Trialkylsilyltrifluormethansulfonate Trimethyl- und Dimethyl-tert.butyl-silyltriflat, als Basen Lithiumdiisopropylamid oder Amine, wie Triethylamin, Pyridin und Imidazol, ein, wobei man gegebenenfalls in einem aprotischen Lösungsmittel, wie Diethylether, Tetrahydrofuran, Dioxan, N,N-Dimethylformamid, Acetonitril oder N-Methylpyrrolidon, arbeitet.

Im Verfahrensschritt c) führt man die Simmon-Smith-Reaktion mit Methylenbromid oder -iodid in Gegenwart von Zn oder Zn-übergangsmetallpaaren, wie Zn/Cu-, Zn/Ag- oder Zn/Co-Paaren, unter Zusatz eines Ethers, wie Diethylether, Diisopropylether oder Dimethoxyethan, und gegebenenfalls eines Lösungsvermittlers, wie Benzol, Toluol oder Methylenchlorid, durch.

Im Verfahrensschritt d) werden für die Birch-Reduktion Alkali- oder Erdalkalimetalle, wie Lithium, Natrium, Kalium oder Calcium, in flüssigem Ammoniak unter Zusatz eines Alkohols, wie Ethanol, n-Propanol, Isopropanol oder tert.Butanol, und gegebenenfalls ein Lösungsvermittler, wie Dioxan oder Tetrahydrofuran, verwendet.

Im Verfahrensschritt e) setzt man für die Enoletherspaltung als Säuren Essigsäure, Oxalsäure, Pyridiniumtosylat, p-Toluolsulfonsäure und verdünnte Schwefelsäure, als wäßrig organische Lösungsmittel wäßriges Aceton, wäßriges Methanol oder ein homogenes Lösungsmittelgemisch, bestehend aus Wasser, Methylenchlorid und tert.Butanol ein.

Im Verfahrensschritt f) wird die Bromierung/Dehydrobromierung mittels Pyridinhydrobromidperbromid oder Brom in Pyridin durchgeführt.

Im Verfahrensschritt g) führt man die Ketalisierung der 3-Ketogruppe in Gegenwart eines wasserentziehenden Mittels, wie Ameisensäuretrimethyl oder Ameisensäuretriethylester, oder in Gegenwart eines Wasserschleppmittels, wie Chloroform, Benzol oder Toluol, durch, und verwendet als Alkohole Methanol, Ethanol, Ethylenglykol oder 2,3-Dimethylpropandiol sowie als Säuren p-Toluolsulfonsäure, Oxalsäure oder Pyridiniumtosylat. Die 17-Alkololfunktion muß vor der Ketalisierung in Form eines Esters oder Dimethyl-tert.butyl-silylethers geschützt werden

Im Verfahrensschritt h) wird die Epoxidierung mit $H_2O_2$ und Chloralhydrat in Gegenwart eines Puffers, sowie von $Na_2HPO_4$, $NaH_2PO_4$, $Na_2CO_3$, $NaHCO_3$, $K_2CO_3$ bzw. von $KHCO_3$ in Form der wasserfreien Salze in Methylenchlorid oder Chloroform durchgeführt.

Im Verfahrensschritt i) wird die Grignardierung in einem Ether, wie Diethylether, Tetrahydrofuran oder Dioxan, gegebenenfalls unter Zusatz eines Lösungsvermittlers, wie Bezol oder Toluol, durchgeführt, weiterhin als Arylmagnesiumhalogenide Phenylmagnesiumhalogenide, die in p-Stellung zum Magnesium eine $OCH_3$-, $SCH_3$-, $N(CH_3)_2$, $NHCH_3$-, CN-, $CH_3CHOH$-, $CH(OR_3)$ $(OR_4)$- oder $CH_3C$-$(OR_3)$ $(OR_4)$-Gruppe enthalten und in denen Halogenid = Bromid oder Chlorid bedeutet, sowie als $Cu^I$-Salze CuCN, CuI und CuCl eingesetzt.

Die folgenden Ausführungsbeispiele dienen der näheren Erläuterung der Erfindung.

Beispiel 1

a) **3,17,17-Trimethoxy-estra-1,3,5(10)-trien**

In einem 100 ml Dreihalskolben werden 5 g 3-Methoxy-estra-1,3,5(10)-trien-17-on (17,5 mmol), 15 ml absolutes Methanol, 0,35 g p-Toluolsulfonsäure und 6 ml Ameisensäure-trimethylester unter Wasserausschluß bei 50 °C ca. 2,5 Stunden gerührt. Nach erfolgter Umsetzung wird das Reaktionsgemisch direkt in die nächste Stufe eingesetzt oder aber nach Abkühlung zur Vervollständigung der Fällung mit Triethylamin und Hexan versetzt. Nach wiederholter Umkristallisation aus Heptan erhält man 5,45 g ( 94 % der Theorie), Schmelzpunkt: 104 °C bis 116 °C.

b) **3,17-Dimethoxy-estra-1,3,5(10),16-tetraen**

Die das 3,17,17-Trimethoxy-estra-1,3,5(10)-trien enthaltende Reaktionslösung wird unter Inertgas innerhalb von 30 - 40 Minuten auf 140 °C bis 160 °C erwärmt, wobei das Lösungsmittel allmählich abdestilliert wird. Zum Schluß wird noch 2 Stunden im Vakuum bei dieser Temperatur erhitzt und der resultierende Enolether in Form des Rohproduktes ohne Reinigung in die nächste Stufe eingesetzt, Schmelzpunkt: 90,5 °C bis 93 °C, IR[$cm^{-1}$]: 1610, 1565, 1490 (Aromat), 1610 (Enol überlagert).

c) **3,17-Dimethoxy-16α,17α-methylen-estra-1,3,5(10)-trien**

10 g 3,17-Dimethoxy-estra-1,3,5(10), 16-tetraen werden in 50 ml Toluen und 40 ml Dimethoxyethan gelöst und nach Zugabe von 20 g Zn/Cu-Katalysator zunächst unter gelindem Erwärmen sukzessive mit 15 ml Methyleniodid unter kräftigem Rühren in einer Inertgasatmosphäre versetzt. Die Methyleniodidzugabe ist dabei so vorzunehmen, daß die Reaktionstemperatur 50 °C nicht übersteigt. Nach dem Abklingen der exothermen Reaktion wird 4 Stunden bei 50 °C gerührt und anschließend vom Katalysator abfiltriert. Das Filtrat wird mit einer wäßrigen Ammoniumchloridlösung versetzt und das Steroid mit Toluen extrahiert. Der nach dem Einengen der Toluenextrakte verbleibende Rückstand wird an basischem Aluminiumoxid mit einem Benzol/n-Hexan-Gemisch (1:1) flash chromatographiert.

Das nach dem Einengen erhältliche Rohprodukt wird aus Hexan kristallisiert. Es werden 6,5 g der 16α,17α-Methylenverbindung erhalten, Schmelzpunkt: 128 °C bis 130 °C, $[\alpha]_D$ = 93,5°.

d) **3,17-Dimethoxy-16α,17α-methylen-estra-2,5(10)-dien**

Zu einer Lösung von 175 ml Tetrahydrofuran und 500 ml flüssigem Ammoniak gibt man unter Schutzgas bei - 50 °C 10 g Natrium, welches in kleine Würfel zerschnitten ist, vorsichtig zu. Nach dem Auflösen des Natriums fügt man 70 ml tert. Butanol hinzu und tropft anschließend 75 ml einer 10 g 3,17-Dimethoxy-16α,17α-methylen-estra-1,3,5(10)-trien enthaltenden Tetrahydrofuranlösung langsam zu. Dann rührt man 4 Stunden bei ca. - 33 °C und versetzt danach mit ca. 10 ml Methanol, wobei die gegebenenfalls noch blaue Lösung entfärbt wird. Man läßt das Ammoniak verdampfen und fällt das Steroid mittels Wasser aus. Nach dem Abfritten und Trocknen werden 10 g des 16α,17α-Methylenenolethers erhalten, die aus Methanol kristallisiert werden können, Schmelzpunkt: 136 °C bis 139 °C, IR[$cm^{-1}$]: 1670 und 1695 (Enolether).

e) **17β-Methoxy-16α,17α-methylen-estr-5(10)-en-3-on**

10 g 3, 17-Dimethoxy-16α,17α-methylen-estra-2,5(10)-dien werden in 165 ml 80prozentigem wäßrigem Aceton suspendiert und unter kräftigem Rühren mit 0,2 ml 25prozentiger Schwefelsäure versetzt. Anschließend wird unter Rühren auf dem Wasserbad (60 °C) erwärmt, bis alles Steroid in Lösung gegangen ist (ca.

30 Minuten). Danach läßt man auf Raumtemperatur abkühlen und rührt noch eine Stunde nach. Nach erfolgter Umsetzung wird das Steroid durch Wasserzugabe ausgefällt, abgetrennt und getrocknet. Man erhält 9,5 g der kristallinen 16α,17α-Methylenverbindung, welche ohne Umkristallisation in die nächste Stufe eingesetzt wurde. IR[$cm^{-1}$]: 1705 (3-Keton).

f) **17β-Methoxy-16α,17α-methylen-estra-4,9-dien-3-on**

9,5 g 17β-Methoxy-16α,17α-methylen-estr-5(10)-en-3-on (Rohprodukt) werden in 130 ml Pyridin gelöst und anschließend unter Kühlung (- 5 °C) mit 11,5 Pyridinhydrobromidperbromid innerhalb von 5 Minuten versetzt. Dann wird die Kühlung weggenommen und die sich langsam auf Raumtemperatur erwärmende Reaktionslösung ca. 30 Minuten gerührt und anschließend mit 3 ml Methylbuten versetzt, wobei überschüssiges Bromierungsmittel verbraucht wird. Dann wird 4 Stunden bei Raumtemperatur gerührt und danach in Eiswasser eingerührt, wobei das Steroid in ölig kristalliner Form ausfällt. Zur vollständigen Kristallisation bewahrt man 16 Stunden im Kühlschrank auf und frittet das Steroid ab. Man erhält 8 g kristallines Rohprodukt. Aus dem Filtrat kann nach Extraktion mit Methylenchlorid und Chromatographie an neutralem Aluminiumoxid mit Benzol/Essigester (20:1 bis 9:1) noch ca. 1 g Dienon erhalten werden, welches aus Aceton/n-Hexan kristallisiert werden kann (Gesamtausbeute 9 g), Schmelzpunkt: 117 °C bis 121 °C, $[\alpha]_D$ = - 203°.

g) **3,3-Ethylendioxy-17β-methoxy-16α,17α-methylen-estra-5(10),9(11)-dien**

8 g 17β-Methoxy-16α,17α-methylen-estra-4,9-dien-3-on (kristallines Rohprodukt) werden in 100 ml Benzen gelöst, mit 6,8 ml Glykol und 0,2 g p-Toluolsulfonsäure versetzt und unter kräftigem Rühren 2 Stunden am Wasserabscheider gekocht. Anschließend wird die abgekühlte Lösung in eine gesättigte Natriumbicarbonatlösung eingerührt und das Steroid mit Benzen extrahiert. Der nach dem Einengen erhältliche Rückstand wird aus Ether/n-Hexan kristallisiert, wobei 7 g des Ketals erhalten werden, Schmelzpunkt: 79 °C bis 84 °C, $[\alpha]_D$ = 220,6°.

h) **3,3-Ethylendioxy-17β-methoxy-16α,17α-methylen-5α,10α-oxido-estr-9(11)-en**

9g (Rohprodukt) 3,3-Ethylendioxy-17β-methoxy-16α,17α-methylen-estra-5(10),9(11)-dien, 2 g wasserfreies $Na_2HPO_4$ und 1 g $Na_2CO_3$ werden in 45 ml Methylenchlorid suspendiert und unter Rühren bei Raumtemperatur mit 7,25 ml 30prozentigem $H_2O_2$ und zuletzt mit 1,25 ml Chloralhydrat versetzt. Dann wird 20 Stunden bei Raumtemperatur gerührt und im Anschluß daran eine wässrige Natrimcarbonatlösung zugegeben und das Steroid mit Methylenchlorid extrahiert. Die organische Phase wird noch zweimal mit einer Natriumcarbonatlösung und zuletzt mit Wasser gewaschen, anschließend getrockent und eingeengt. Der ölige Rückstand wird an basischem $Al_2O_3$ chromatographiert mit Benzol/Essigester (20:1 bis 9:1). Man erhält 6,6 g 5α,10α-Epoxid in Form eines Öles.

i) **11β-(4-Dimethylaminophenyl)-3,3-ethylendioxy-17β-methoxy-16α,17α-methylen-estr-9-en-5α-ol**

30 ml einer Grignardlösung, bereitet aus 0,96 g Magnesium, 5 ml Tetrahydrofuran, 0,05 ml Dibromethan und 8,4 g p-Brom-dimethylaminobenzol in 55 ml Tetrahydroduran, werden auf ca. - 15 °C abgekühlt und mit 0,25 g CuCl versetzt. Nach 15 Minuten werden in der Kälte ca. 2,34 g 3,3-Ethylendioxy-17β-methoxy-16α,17α-methylen-5α,10α-oxido-estr-9(11)-en, die in 10 ml Tetrahydrofuran gelöst sind, zugetropft, Danach läßt man langsam auf Raumtemperatur erwärmen. Nach einer Stunde ist alles Ausgangsmaterial umgesetzt. Es wird mit wässriger Ammoniumchloridlösung versetzt und das Steroid mit Ether extrahiert. Die organische Phase wird mit Wasser gewaschen, anschließend getrocknet und eingeengt. Nach Chromatographie an basischem Aluminiumoxid, die Elution erfolgt mit Benzen/Essigester (20:1 bis 9:1), erhält man 2,29 g der 11β-Dimethylaminophenylverbindung, welche aus Methanol kristallisiert werden kann, Schmelzpunkt 151 °C bis 156 °C, $[\alpha]_D$ = 43,6 °.

j) **11β-(4-Dimethylaminophenyl)-17β-methoxy-16α,17α-methylen-estra-4,9-dien-3-on**

0,4 g 11β-(4-Dimethylaminophenyl)-3,3-ethyledioxy-17β-methoxy-16α,17α-methylen-estr-9-en-5α-ol werden in 7 ml 70prozentiger wässriger Essigsäure gelöst und 1 Stunde bei 60 °C Wasserbadtemperatur unter Rühren erwärmt. Nach erfolgter Umsetzung wird in kaltes Wasser eingerührt, das etwas Ammoniak zur Neutralisation der Säure enthält. Das ausgefallene Produkt wird abgetrennt und an neutralem Aluminiumoxid flash chromatographiert mit einem Benzol/Essigester-Gemisch (10:1). Nach Kristallisation aus Methanol/Wasser erhält man 0,3 g der Dimethylaminophenylverbindung, Schmelzpunkt: 87 °C bis 91 °C, $[\alpha]_D$ = 295,2 °.

Beispiel 2

Die Herstellung der Reaktionsstufen a bis h erfolgt analog zu Beispiel 1.

a) **3,3-Ethylendioxy-17β-methoxy-16α,17α-methylen-11β-[4-(2'-methyl-1',3'-dioxolan-2'yl-)phenyl]-estr-9-en-5α-ol**

Zu einer Suspension von 0,72 g Magnesiumspänen in 5 ml Tetrahydrofuran fügt man 0,05 ml Dibromethan hinzu und versetzt unter Argon sukzessive mit 55 ml einer 7,35 g p-Brom-(2'-methyl-1',3'-dioxolan-2'yl)-benzol enthaltenden Tetrahydrofuranlösung so zu, daß die Innentemperatur 45 °C nicht übersteigt. In der Startphase wird leicht erwärmt (45 °C) und nachdem der Grignard angesprungen ist, die Temperatur durch die Zugabe des Arylhalogenids geregelt. Nach erfolgter Zugabe wird noch 2 Stunden bei 45 °C gerührt. Von der so bereiteten Grignardlösung werden 37 ml entnommen und unter Kühlung (- 5 °C bis - 15 °C) mit 0, 15 g CuCl versetzt. Man rührt 15 Minuten unter Beibehaltung dieser Temperatur und fügt dann in der Kälte eine Lösung von 2 g 3,3- Ethylendioxy-17β-methoxy-16α,17α-methylen-5α,10α-oxido-estr-9(11)-en (1 h) in 10 ml Tetrahydrofuran so zu, daß sich die Lösung nicht wesentlich erwärmt. Nach erfolgter Umsetzung wird mit einer wäßrigen Ammoniumchloridlösung versetzt und das Steroid mit Ether extrahiert. Nach dem Einengen wird der verbleibende Rückstand an basischem Alumiunoxid chromatographiert mit Benzol/Essigester (20:1 bis 9:1). Man erhält 1,5 g der 11β-Arylverbindung, welche aus Ether/n-Hexan kristallisiert werden kann, Schmelzpunkt: 137 °C bis 144 °C, $[\alpha]_D$ = 31,2 °.

j) **11β-(4-Acetylphenyl)-17β-methoxy-16α,17α-methylen-estra-4,9-dien-3-on**

0,45 g 3,3-Ethylendioxy-17β-methoxy-16α,17α-methylen-11β-[4-(2'-methyl-1',3')-dioxolan-2'-yl)-phenyl]-estr-9-en-5α-ol werden in 10 ml 70prozentiger wäßriger Essigsäure gelöst und auf dem Wasserbad bei 60 °C ca. 1 Stunde gerührt. Nach erfolgter Umsetzung wird die Lösung in der Kälte mit Wasser versetzt, wobei 0,3 g des Steroids in amorph kristalliner Form ausgefällt werden. Nach Chromatographie an basischem Aluminiumoxid mit Benzol/Essigester (20:1), werden 0,2 g des 4,9-Diens erhalten, welches aus Methanol/Wasser kristallisiert wird, Schmelzpunkt: 84 °C bis 87 °C, $[\alpha]_D$ = 279,2 °.

Beispiel 3

Die Herstellung der Reaktionsstufen a bis h erfolgt analog zu Beispiel 1.

i) **11β-[4-(Diethoxymethyl)-phenyl]-3,3-ethylendioxy-17-methoxy-16α,17α-methylen-estr-9-en-5α-ol**

30 ml (15 mmol) einer p-Diethoxymethylphenylmagnesiumbromid-Lösung in Tetrahydrofuran, bereitet aus 0,72 g Magnesiumspänen, 7 ml (8,85 g, 30 mmol) p-Brom-diethoxymethylbenzen in 60 ml Tetrahydrofuran und 0,05 ml Dibromethan bei einer maximalen Temperatur von 45 °C, werden mittels Trockeneis auf ca. - 15 °C abgekühlt und mit 0,2 g CuCl versetzt. Nach 15minütigem Rühren wird in der Kälte 1g (Öl; 2,79 mmol) 3,3-Ethylendioxy-17β-methoxy-16α,17α-methylen-5α,10α-oxido-estr-9(11)-en, welches in 10 ml Tetrahydrofuran gelöst ist, zugetropft. Anschliessend wird 1 Stunde unter Feuchtigkeitsausschluß gerührt, wobei sich die Lösung auf Raumtemperatur erwärmt. Dann wird mit einer wäßrigen Ammoniumchloridlösung versetzt und das Steroid mit Ether extrahiert. Nach dem Einengen der Extrakte wird der verbleibende Rückstand an basischem Aluminiumoxid chromatographiert mit Benzol/Essigester (10:1). Es wurden 0,65 g der Zielverbindung in Form eines Öles isoliert.
IR[$cm^{-1}$]: 1600 (Aromat), 3500 (OH, assoziiert).

j) **11β-(4-Formylphenyl)-17β-methoxy-16α,17α-methylen-estra-4,9-dien-3-on**

0,6 g 11β-[4-(Diethoxymethyl)-phenyl]-3,3-ethylendioxy-17β-methoxy-16α,17α-methylen-estr-9-en-5α-ol werden in 10 ml 70prozentiger wässriger Essigsäure gelöst und ca. 2 Stunden bei 70 °C auf dem Wasserbad erwärmt. Dann wird das Stereoid durch Zugabe von Wasser und etwas Ammoniak ausgefällt. Das isolierte Rohprodukt wird an neutralem Aluminiumoxid flash chromatographiert mit Benzol/Essigester (10:1). Man erhält 0,41 g der Zielverbindung, welche aus Ether oder Acetonitril kristallisiert werden kann, Schmelzpunkt: 196 °C bis 199,5 °C, $[\alpha]_D$: 315,9 °.

Beispiel 4

Die Herstellung der Reaktionsstufen a bis h erfolgt analog zu Beispiel 1.

i) **3,3-Ethylendioxy-11-β-(4-formylphenyl)-17β-methoxy-16α,17α-methylen-estr-9-en-5α-ol**

Beim Versuch, obiges Grignardprodukt (Beispiel 3i) über Kieselgel (präparative Platte, Laufmittel Benzen/Essigester 5:1) feinzureinigen, wird das Diethylacetat gespalten und es entsteht das 11β-(4-Formylphenyl)-3-ketal, das aus Aceton oder Methanol kristallisiert werden kann, Schmelzpunkt: 184 °C bis 187 °C, $[\alpha]_D$ = - 198,6 °.

Beispiel 5

a) **17,17-Diethoxy-3-methoxy-estra-1,3,5(10)-trien**

5 g 3-Methoxy-estra-1,3,5(10)-trien-17-on werden in 15 ml absolutem Ethanol, 15 ml Benzen und 6 ml Ameisensäuretriethylester gelöst und nach Zugabe von 0,35 g p-Toluensulfonsäure 2,5 Stunden bei 50 °C gerührt. Nach erfolgter Umsetzung wird das Reaktionsgemisch entweder direkt in die nächste Stufe eingesetzt oder aber in eine wässrige Natriumbicarbonatlösung eingerührt und das Steroid mit Benzen extrahiert. Die Extrakte werden zur Trockenen eingeengt und aus Ethanol kristallisiert, wobei 4,8 g Diethylketal erhalten werden, Schmelzpunkt: 93 °C bis 97 °C.

b) **17β-Ethoxy-3-methoxy-estra-1,3,5(10),16-tetraen**

5 g 17,17-Diethoxy-3-methoxy-estra-1,3,5(10)-trien werden in 20 ml Mesitylen gelöst und nach Zugabe von 0,3 g $KHSO_4$ unter Inertgas innerhalb von 30 Minuten bis 40 Minuten auf 140 °C bis 160 °C erwärmt, wobei vom Lösungsmittel allmählich abdestilliert wird. Dann wird noch 2 Stunden im Vakuum bei dieser Temperatur erhitzt und das restliche Lösungsmittel abdestilliert. Der verbleibende Rückstand wird in Form eines Öles ohne Reinigung in die nächste Stufe eingesetzt, IR[$cm^{-1}$]: 1500, 1575, 1620 (Aromat); 1620 (Enolether überlagert).

c) **17β-Ethoxy-3-methoxy-16α,17α-methylen-estra-1,3,5(10)-trien**

2,5 g 17β-Ethoxy-3-methoxy-estra-1,3,5(10),16-tetraen werden in 5 ml Benzen und 5 ml Dimethoxyethan gelöst und unter Inertgas mit 5g Zn/Cu-Katalysator (Le Goff) versetzt. Zu diesem Reaktionsgemisch wird unter gelindem Erwärmen und kräftigem Rühren sukzessive eine Lösung bestehend aus 4,5 ml Methyleniodid und 4 ml Benzen so zugetropft, daß die Reaktionstemperatur 50 °C nicht übersteigt. Nach dem Abklingen der exothermen Reaktion, gegebenenfalls muß bei der Zugabe das Wärmebad entfernt werden, wird 4 Stunden bei 50 °C gerührt und anschließend vom Katalysator abfiltriert. Das Filtrat wird mit einer wässrigen Ammoniumchloridlösung versetzt und das Steroid mit Benzen extrahiert. Der nach dem Einengen der Benzenextrakte verbleibende Rückstand wird an neutralem Aluminiumoxid flash chromatographiert mit Benzol/n-Hexan (1:1). Man erhält 1,7 g (65% der Theorie) der 16α,17α-Methylenverbindung, die aus Methanol umkristallisiert werden kann, Schmelzpunkt: 89 °C bis 91,5 °C, $[\alpha]_D$ = 84 °.

d) **17β-Ethoxy-3-methoxy-16α,17α-methylen-estra-2,5(10)-dien**

Die Herstellung erfolgte entsprechend Beispiel 1d. Das semikristalline Rohprodukt wurde direkt in die nächste Stufe eingesetzt.

e) **17β-Ethoxy-16α,17α-methylen-estr-5(10)-en-3-on**

Die Herstellung erfolgte entsprechend Beispiel 1e.
Schmelzpunkt Methanol: 94 °C bis 96 °C, $[\alpha]_D$ = 174 °.

f) **17β-Ethoxy-16α,17α-methylen-estra-4,9-dien-3-on**

Die Herstellung erfolgte entsprechend Beispiel 1f.
Schmelzpunkt Ether/Hexan: 104,5 °C bis 106,5 °C, $[\alpha]_D$ = - 160 °.

g) **17β-Ethoxy-3,3-ethylendioxy-16α,17α-methylen-estra-5(10),9(11)-dien**

Die Herstellung erfolgte entsprechend Beispiel 1g.
Schmelzpunkt Hexan: 117,5 °C bis 118 °C, $[\alpha]_D$ = 207 °.

h) **17β-Ethoxy-3,3-ethylendioxy-16α,17α-methylen-5α,10α-oxido-estr-9(11)-en**

6 g 17β-Ethoxy-3-3-ethylendioxy-16α,17α-methylen-estr-5(10), 9(11)-dien, 2g wasserfreies $Na_2HPO_4$ und 1 g $Na_2CO_3$ werden in 30 ml Methylenchlorid suspendiert und unter Rühren bei Raumtemperatur mit 3,5 ml 30prozentigem $H_2O_2$ und zuletzt mit 1 g Chloralhydrat versetzt. Dann wird 20 Stunden bei Raumtemperatur gerührt, anschließend mit einer wäßrigen Natriumcarbonatlösung versetzt und das Steroid mit Methylenchlorid extrahiert. Die organische Phase wird noch zweimal mit einer Natriumcarbonatlösung und zuletzt mit Wasser gewaschen, anschließend getrocknet und eingeengt. Man erhält 6,074 g eines langsam kristallisierenden Rohproduktes, das aus Hexan umkristallisiert werden kann. Schmelzpunkt: 160 °C bis 170 °C.

i) **17β-Ethoxy-3,3-ethylendioxy-11β-(4-methoxyphenyl)-16α,17α-methylen-estr-9-en-5α-ol**

Von einer durch Umsetzung von 0,48 g Magnesiumspänen und 2,36 ml 4-Bromanisol in 20 ml Tetrahydrofuran bei 35 °C dargestellten 4-Methoxyphenylmagnesiumbromidlösung werden 20 ml entnommen und unter Argon und Kühlung auf - 5 °C bis - 15 °C mit 0,1 g CuCl versetzt. Man rührt 15 Minuten unter Beibehaltung der Kühlung und fügt dann eine Lösung von 1 g 17β-Ethoxy-3,3-ethylendioxy-16α,17α-methylen-5α,10α-oxido-estr-9(11)-en in 3 ml Tetrahydrofuran tropfenweise hinzu. Anschließend wird 30 Minuten bei Raumtemperatur gerührt, danach eine wässrige Ammoniumchloridlösung zugesetzt und das Steroid mit Methylenchlorid extrahiert. Nach dem Einengen der Extrakte wird der Rückstand an basischem Aluminiumoxid (Greiz-Dölau) flash chromatographiert mit Benzol/Essigester (20:1). Nach Umkristallisation aus Methanol erhält man 0,37 g (27 % der Theorie) der 11β-Anisylverbindung. Schmelzpunkt: 144 °C bis 147 °C, $[\alpha]_D$ = 180,4 °.

j) **17β-Ethoxy-11β-(4-methoxyphenyl)-16α,17α-methylen-estra-4,9-dien-3-on**

0,5 g 17β-Ethoxy-3,3-ethylendioxy-11β-(4-methoxyphenyl)-estr-9-en-5α-ol werden in 7 ml 70prozentiger wäßriger Essigsäure gelöst und ca. 2 Stunden bei 60 °C Wasserbadtemperatur erwärmt. Nach erfolgter Umsetzung wird das Steroid durch Zugabe von Wasser ausgefällt und nach Abtrennung an neutralem Aluminiumoxid flash chromatographiert mit Benzol/Essigester (10:1). Es werden 0,2 g des 11β-Anisyl-4,9-diens erhalten, welches aus Methanol kristallisiert werden kann. Schmelzpunkt: 73,5 °C bis 74,5 °C, $[\alpha]_D$ = 210,7.

Beispiel 6

Die Herstellung der Reaktionsstufen a bis h erfolgt analog zu Beispiel 5.

i) **11β-(4-Dimethylaminophenyl)-17β-ethoxy-3,3-ethylendioxy-16α,17α-methylen-estr-9-en-5α-ol**

Zu einer Suspension von 0,48 g Magnesiumsspänen in 10 ml Tetrahydrofuran fügt man 0,05 ml Methyliodid hinzu und versetzt unter Argon sukzessive mit einer Lösung von 4,2 g p-Brom-dimethylaminobenzol in 30 ml Tetrahydrofuran, wobei die Innentemperatur 50 °C nicht übersteigen sollte. Von der so erzeugten p-Dimethylaminophenylmagnesiumbromidlösung entnimmt man 24 ml und versetzt unter Kühlung (-15 °C) mit 0, 15 g CuCl. Man rührt etwa 15 Minuten unter Beibehaltung dieser Temperatur und fügt eine Lösung von 0,75 g eines mit 17β-Ethoxy-3,3-ethylendioxy-16α,17α-methylen-5α,10α-oxido-estr-9(11)-en angereicherten Epoxids in 4 ml Tetrahydrofuran tropfenweise hinzu. Anschließend wird 4 Stunden bei einer Temperatur von etwa 0 °C gerührt, danach eine wässrige Ammoniumchloridlösung zugesetzt und das Steroid mit Methylenchlorid exrahiert. Nach dem Einengen der Extrakte wird der Rückstand ans basischem Aluminiumoxid (Greiz-Dölau) flash chromatographiert. Als mobile Phase dient Benzol/Essigester (10:1). Man erhält 0,3 g der 11β-Dimethylaminphenylverbindung, die aus Methanol umkristallisiert werden kann. Schmelzpunkt: 117 °C bis 119 °C, $[\alpha]_D$ = 31,8 0.

j) **11β-(4-Dimethylaminophenyl)-17β-ethoxy-16α,17α-methylen-estra-4,9-dien-3-on**

Die Herstellung erfolgt entsprechend Beispiel 1 Stufe j.
Schmelzpunkt: 84 °C bis 87 °C, $[\alpha]_D$ = 355,7 °.

Beispiel 7

Die Herstellung der Reaktionsstufen a bis h erfolgt analog zu Beispiel 5.

**i) 17β-Ethoxy-3,3-ethylendioxy-16α,17α-methylen-11β-[4-(2'-methyl-1',3'-dioxolan-2'-yl-)phenyl]-estr-9-en-5α-ol**

Zu einer Suspension von 0,48 g Magnesiumspäne in 13 ml THF fügt man 0,05 ml Methyliodid hinzu und versetzt unter Argon sukzessive mit einer Lösung von 4,9 g 4-Brom-(2'-methyl-1,3-dioxolan-2'-yl-)benzen in 27 ml THF, wobei die Innentemperatur 45° C nicht übersteigen sollte. Nach Auflösung des Magnesiums entnimmt man 40 ml 4-(2'-Methyl-1',3'-dioxolan-2'-yl-)phenyl-magnesiumbromid und gibt dazu unter Kühlung (-5° C bis -15° C) 0,2 g CuCl. Man rührt 15 Minuten unter Beibehaltung dieser Temperatur und fügt dann eine Lösung von 1,69 g 17β-Ethoxy-3,3-ethylendioxy-16α,17α-methylen-5α,10α-oxido-estr-9(11)-en in 5 ml THF tropfenweise hinzu. Anschließend wird 1 Stunde gerührt, wobei die Reaktionslösung allmählich auf Raumtemperatur gebracht wird. Nach erfolgter Umsetzung wird eine wäßrige Ammoniumchloridlösung zugesetzt und das Steroid mit Methylenchlorid extrahiert. Nach dem Einengen der Extrakte wird der verbleibende Rückstand an basischem Aluminiumoxid (Greiz-Dölau) chromatographiert. Als mobile Phase wird ein Benzol/Essigester-Gemisch (20:1) verwendet. Man erhält 1,7 g der 11β-Acetophenylketalverbindung, die aus Methanol umkristallisiert werden kann.

Schmelzpunkt 95° C bis 97° C $[\alpha]_D$: 22,5°

**11β-(4-Acetylphenyl)-17β-ethoxy-16α,17α-methylen-estra-4,9-dien-3-on**

Die Herstellung erfolgt entsprechend Beispiel 2 Stufe j.

Schmelzpunkt Methanol/Wasser: 86° C bis 90° C $[\alpha]_D$: 280°

Beispiel 8

Die Herstellung der Reaktionsstufen a bis h erfolgt analog zu Beispiel 5.

**i) 11β-[4-(Diethoxymethyl)phenyl]-17β-ethoxy-3,3-ethylendioxy-16α,17α-methylen-estr-9-en-5α-ol**

1 g (2,69 mmol) 17β-Ethoxy-3,3-ethylendioxy-16α,17α-methylen-5α,10α-oxido-estr-9(11)-en werden analog Beispiel 3 Stufe i zur Umsetzung gebracht. Nach Aufarbeitung und chromatographischer Reinigung, die ebenfalls in der schon beschriebenen Weise erfolgten, wurden 0,9 g des Grignardproduktes in Form eines Öles isoliert.

IR[$cm^{-1}$]: 1600 (Aromat), 3500 (OH - assoziiert)

**j) 17β-Ethoxy-11β-(4-formylphenyl)-16α,17α-methylen-estra-4,9-dien-3-on**

Die Herstellung erfolgt entsprechend Beispiel 3 Stufe j.

Schmelzpunkt 79° C bis 84° C $[\alpha]_D$: 411,1°

Beispiel 9

Die Herstellung der Reaktionsstufen a bis h erfolgt analog zu Beispiel 5.

**i) 17β-Ethoxy-3,3-ethylendioxy-16α,17α-methylen-11β-vinyl-estr-9-en-5α-ol**

10 ml einer 0,5 m Lösung Vinylmagnesiumbromid in Tetrahydrofuran werden auf -30° C heruntergekühlt und unter Inertgas mit 0,1 g CuCl versetzt. Man rührt ca. 15 Minuten bei dieser Temperatur und tropft anschließend 5 ml einer 0,3 g 17β-Ethoxy-3,3-ethylendioxy-16α,17α-methylen-5α,10α-oxido-estr-9-en enthaltende Tetrahydrofuranlösung in der Kälte zu. Dann wird 8 Stunden bei maximal -15° C gerührt. Nach erfolgter Umsetzung wird mit wäßriger Ammoniumchloridlösung versetzt und das Steroid mit Ether extrahiert. Nach Chromatographie an basischem Aluminiumoxid, als Elutionsmittel wird ein Benzen/Essigester-Gemisch (10:1) verwendet, werden 0,25 g der 11β-Vinylverbindung in Form eines Öles isoliert, die direkt in die nächste Stufe eingesetzt werden.

IR[$cm^{-1}$]: 1625 (Vinyl), 3500 (OH - assoziiert)

j) **17β-Ethoxy-16α,17α-methylen-11β-vinyl-estra-4,9-dien-3-on**

Die Herstellung erfolgt entsprechend Beispiel 1 Stufe j. $^1$H-NMR[ppm]: 5,61 (1H, 4-en) , 4,95 u. 4,77 (3H, Vinyl) 1,14; 1,07; 1,00 (Me v. O $C_2H_5$) , 1,03 (3H, 13-Me) , 0,6 (2H, Cyclopropan) IR[$cm^{-1}$]: 1600 und 1660 (Dienon)

Beispiel 10

Die Herstellung der Reaktionsstufen a bis h erfolgt zu Beispiel 5 und die der Stufen i und j analog Beispiel 1.

k) **11β-(4-Dimethylaminophenyl)-17β-ethoxy-3,3-ethylendithio-16α,17α-methylen-estra-4,9-dien**

0,065 g 11β-(4-Dimethylaminophenyl-17β-ethoxy-16α,17α-methylen-estra-4,9-dien-3-on werden in 0.03 ml Methanol gelöst und nach Zugabe von 0,03 ml Thioglykol und 0,03 ml Bortrifluoridetherat 2,5 Stunden bei Raumtemperatur gerührt. Anschließend wird mit Wasser versetzt und das Steroid mit Ether extrahiert. Nach Fällung aus Methanol/Wasser/Ammoniak werden 0,063 g des amorphen Thioketals isoliert.
Schmelzpunkt: 109 °C bis 114 °C, $[\alpha]_D$ = 262,6 °.

Beispiel 11

a) **17,17-Di-n-butyloxy-3-methoxy-estra-1,3,5(10)-trien**

5 g 3-Methoxy-estra-1,3,5(10)-trien-17-on werden entsprechend Beispiel 1a zur Umsetzung gebracht. Als Alkohol wird n-Butanol verwendet. Nach erfolgter Umsetzung wird das Reaktionsgemisch ohne Zwischenaufarbeitung direkt in die nächste Stufe eingesetzt.

b) **17-n-Butyloxy-3-methoxy-estra-1,3,5(10),16-tetraen**

Das bei der Synthese von Di-n-butyloxy-3-methoxy-estra-1,3,5(10)-trien erhaltene Reaktionsgemisch wird mit 3 ml Triethylamin versetzt und unter Inertgas innerhalb von 30 - 40 Minuten auf 140 °C bis 160 °C erwärmt, wobei das Lösungsmittel allmählich abdestilliert wird. Zum Schluß wird noch 2 Stunden im Vakuum bei dieser Temperatur erhitzt und restliches Lösungsmittel entfernt. Der verbleibende Rückstand wird nach Abkühlung mit Natriumbicarbonatlösung versetzt und das Steroid mit Benzen extrahiert. Nach Chromatographie an basischem Aluminiumoxid, als Elutionsmittel dient Benzen/Hexan, erhält man 3,95 (66 % der Theorie) des Tetraens, welches in Form eines Öles weiterverarbeitet wird. IR[$cm^{-1}$]: 1575 und 1620 (Aromat), 1620 (Enolether überlagert).

c) **17β-n-Butyloxy-3-methoxy-16α,17α-methylen-estra-1,3,5(10)-trien**

Die Herstellung erfolgt entsprechend Beispiel 1c.
Schmelzpunkt Ethanol: 71 °C bis 72 °C, $[\alpha]_D$ = 80,1 °.

d) **17β-Butyloxy-3-methoxy-16α,17α-methylen-estra-2,5(10)-dien**

Die Herstellung erfolgt entsprechend Beispiel 1d.
IR[$cm^{-1}$]: 1680 und 1700 (Enolether).

e) **17β-Butyloxy-16α,17α-methylen-est-5(10)-en-3-on**

Die Herstellung erfolgt entsprechend Beispiel 1e.
IR[$cm^{-1}$] : 1705 (3-Keton).

f) **17β-Butyloxy-16α,17α-methylen-estra-4,9-dien-3-on**

Die Herstellung erfolgt entsprechend Beispiel 1f.
IR[$cm^{-1}$]: 1600 und 1655 (Dienon).

g) **17β-n-Butyloxy-3,3-ethylendioxy-16α,17α-methylen-estra-5(10),9(11)-dien**

Die Herstellung erfolgt entsprechend Beispiel 1g.
IR[$cm^{-1}$]: kein Carbonyl.

h) **17β-Butyloxy-3,3-ethylendioxy-16α,17α-methylen-5α,10α-oxido-estr-9(11)-en**

Die Herstellung erfolgt entsprechend Beispiel 1h.
IR[$cm^{-1}$]: kein Carbonyl.

i) **17β-n-Butyloxy-11β-(4-dimethylaminophenyl)-3,3-ethylendioxy-est-9-en-5α-ol**

Die Herstellung erfolgt entsprechend Beispiel 1i.
Schmelzpunkt Methanol: 147 °C bis 151 °C, $[\alpha]_D$ = 22,3 °.

j) **17β-n-Butyloxy-11-β-(4-dimethylaminophenyl)-16α,17α-methylen-estra-4,9-dien-3-on**

Die Herstellung erfolgt entsprechend Beispiel 1j.
Schmelzpunkt Methanol/Wasser: 63 °C bis 68 °C, $[\alpha]_D$ = 283,6 °.

Beispiel 12

Die Herstellung der Reaktionsstufen a bis h erfolgt analog zu Beispiel 11.

i) **17β-Butyloxy-3,3-ethylendioxy-16α,17α-methylen-11β-[4-(2'-methyl-1',3'-dioxolan-2'-yl)-phenyl]-estr-9-en-5α-ol**

Die Herstellung erfolgt entsprechend Beispiel 2i.
Schmelzpunkt: 77 °C bis 80 °C, $[\alpha]_D$ = 15 °.

j) **11β-(4-Acetylphenyl)-17β-n-butyloxy-16α,17α-methylen-estra-4,9-dien-3-on**

0,39 g 17β-Butyloxy-3,3-ethylendioxy-16α,17α-methylen-11β-[4-(2'-methyl-1',3'dioxolan-2'-yl)-phenyl]-estr-9-en-5α-ol werden in 10 ml 70prozentiger wäßriger Essigsäure gelöst und auf dem Wasserbad bei 60°C ca. 1 Stunde gerührt. Nach erfolgter Umsetzung wird die Lösung in der Kälte mit Wasser versetzt, wobei das Steroid in amorph kristalliner Form ausgefällt und abgefrittet werden kann. Nach Chromatographie an basischem Aluminiumoxid mit Benzol/Essigester (20:1 bis 9:1) werden neben geringen Mengen eines unpolaren Produkts (11β-(4-Acetylphenyl)-17β-butyloxy-16α,17α-methylen-estra-5(10),9(11)-dien-3-on) ca. 0,25 g des 4,9-Dien3-ons nach Fällung mittels Methanol/Wasser in Form eines amorphen Pulvers erhalten.
Schmelzpunkt: 63 °C bis 67 °C, $[\alpha]_D$ = 171,8 °.

Beispiel 13

a) **17,17-Di-n-hexyloxy-3-methoxy-estra-1,3,5(10)-trien**

5 g 3-Methoxy-estra-1,3,5(10)-trien-17-on werden entsprechend Beispiel 1a zur Umsetzung gebracht. Als Alkohol wird n-Hexanol verwendet. Das Reaktionsgemisch wurde ohne vorherige Aufarbeitung in die nächste Stufe eingesetzt.

b) **n-Hexyloxy-3-methoxy-estra-1,3,5(10),16-tetraen**

Die Herstellung erfolgt entsprechend Beispiel 1b.
IR[$cm^{-1}$]: 1575 und 1620 (Aromat), 1620 (Enolether, überlagert).

c) **17β-n-Hexyloxy-3-methoxy-16α,17α-methylen-estra-1,3,5(10)-trien**

Die Herstellung erfolgt entsprechend Beispiel 1c.
Schmelzpunkt: 38 °C bis 41 °C, $[\alpha]_D$ = 67,7 °.

d) **17β-n-Hexyloxy-3-methoxy-16α,17α-methylen-estra-2,5(10)-dien**

Die Herstellung erfolgt entsprechend Beispiel 1d.
IR[$cm^{-1}$]: 1655 und 1680 (Enolether).

e) **17β-n-Hexyloxy-16α,17α-methylen-estr-(5(10)-en-3-on**

Die Herstellung erfolgt ensprechend Beispiel 1e.
IR[$cm^{-1}$]: 1705 (C=0).

f) **17β-n-Hexyloxy-16α,17α-methylen-estra-4,9-dien-3-on**

Die Herstellung erfolgt ensprechend Beispiel 1f.
Schmelzpunkt Methanol: 57 °C bis 61 °C, $[\alpha]_D$ = - 163,3 °.

g) **3,3-Ethylendioxy-17-n-hexyloxy-16α,17α-methylen-estra-5(10),9(11)-dien**

Die Herstellung erfolgt ensprechend Beispiel 1g.
IR[$cm^{-1}$]: kein Carbonyl.

h) **3,3-Ethylendioxy-17β-n-hexyloxy-16α,17α-methylen-5α,10α-oxido-estr-9(11)-en**

Die Herstellung erfolgt ensprechend Beispiel 1h.
IR[$cm^{-1}$] : kein Carbonyl.

i) **17β-n-Hexyloxy-3,3-ethylendioxy-16α,17α-methylen-11β-[4-(2'-methyl-1',3'-dioxolan-2'-yl)-phenyl]-estr-9-en-5α-ol**

Die Herstellung erfolgt ensprechend Beispiel 2i.
IR[$cm^{-1}$]: 1500 und 1605 (Aromat), 3500 (OH - assoziiert).

j) **11β-(4-Acetylphenyl)-17β-n-hexyloxy-16α,17α-methylen-estra-4,9-dien-3-on**

Die Herstellung erfolgt ensprechend Beispiel 2j.
Schmelzpunt Methanol: 53 °C bis 58 °C.

Beispiel 14

a) **17,17-Di-methoxyethyloxy-3-methoxy-estra-1,3,5(10)-trien**

5 g 3-Methoxy-estra-1,3,5(10)-trien-17-on werden entsprechend Beispiel 1a zur Umsetzung gebracht. Als Alkohol wird Ethylenglykolmonomethylether verwendet. Das Reaktionsgemisch wurde ohne vorherige Aufarbeitung direkt in die nächste Stufe eingesetzt.

b) **3-Methoxy-17β-methoxyethyloxy-estra-1,3-5(10),16-tetraen**

Die Herstellung erfolgt ensprechend Beispiel 1b.
IR[$cm^{-1}$]: 1575 und 1620 (Aromat), 1620 (Enolether überlagert).

c) **3-Methoxy-17β-methoxyethyloxy-16α,17α-methylen-estra-1,3,5(10)-trien**

Die Herstellung erfolgt ensprechend Beispiel 1c.
Schmelzpunkt: 86,5 °C bis 89 °C, $[\alpha]_D$ = 78,6 °.

d) **3-Methoxy-17β-methoxyethyloxy-16α,17α-methylen-estra-2,5(10)-dien**

Die Herstellung erfolgt ensprechend Beispiel 1d.
Schmelzpunkt Hexan: 80 °C bis 96 °C, $[\alpha]_D$ = 116,6 °.

e) **17β-Methoxyethyloxy-16α,17α-methylen-estr-5(10)-en-3-on**

Die Herstellung erfolgt ensprechend Beispiel 1e.
IR[$cm^{-1}$]: 1705 (C=O).

f) **17β-Methoxyethyloxy-16α,17α-methylen-estra-4,9-dien-3-on**

Die Herstellung erfolgt ensprechend Beispiel 1f.
Schmelzpunkt Hexan: 88,5 °C bis 90,5 °C, $[\alpha]_D$ = - 160,2 °.

g) **1,3-Ethylendioxy-17β-methoxyethyloxy-16α,17α-methylen-estra-5(10),9(11)** -dien

Die Herstellung erfolgt ensprechend Beispiel 1g.
IR[$cm^{-1}$]: kein C=O.

h) **3,3-Ethylendioxy-17β-methoxyethyloxy-16α,17α-methylen-5α,10α-oxido-estr-9(11)-en**

Die Herstellung erfolgt entsprechend Beispiel 1h.
IR[$cm^{-1}$]: kein C=O.

i) **3,3-Ethylendioxy-17β-methoxyethyloxy-16α,17α-methylen-11β-[4-(2'-methyl-1',3'-dioxolan-2'-yl)-phenyl]-estr-9-en-5α-ol**

Die Herstellung erfolgt entsprechend Beispiel 2i.
IR[$cm^{-1}$]: 1475 und 1595 (Aromat), 3480 (OH - assoziiert).

j) **11β-(4-Acetylphenyl)-17β-methoxyethyloxy-16α,17α-methylen-estra-4,9-dien-3-on**

Die Herstellung erfolgt entsprechend Beispiel 2j.
Schmelzpunkt Methanol/Wasser: 70 °C bis 73,5 °C, $[\alpha]_D$ = 248,3 °.

<u>Beispiel 15</u>

a) **17-(Dimethyl-tert.butyl-silyloxy)-3-methoxy-estra-1,3,5(10),16-tetraen**

1,5 g 3-Methoxy-estra-1,3,5(10)-trien-17-on (5,27 mmol) werden unter Feuchtigkeitsausschluß unter Inertgas in 5 ml über $P_2O_5$ destilliertem Methylenchlorid gelöst und mit 2,4 g Trifluormethansulfonsäure-dimethyl-tert.butylsilylester und 1,5 ml Triethylamin (KOH getrocknet) zur Umsetzung gebracht. Dann wird 12 Stunden bei Raumtemperatur stehen gelassen und nach erfolgter Umsetzung das Lösungsmittel im Vakuum abdestilliert. Der verbleibende Rückstand wird an basischem Aluminiumoxid chromatographiert. Als mobile Phase dient Petrolether. Man erhält 2,07 g (98 % d. Th.) Silylenolether, welcher aus n-Hexan kristallisiert werden kann.
Schmelzpunkt: 123°C bis 124,5°C.

b) **17β-(Dimethyl-tert.butyl-silyloxy)-3-methoxy-16α,17α-methylen-estra-1,3,5(10)-trien**

1,6 g 17-(Dimethyl-tert.butyl-silyloxy)-3-methoxy-estra-1,3,5(10),16-tetraen werden unter Feuchtigkeitsausschluß unter Rühren zu einer Suspension von 10 g Zn/Cu-Katalysator in 15 ml Dimethoxyethan und 15 ml Diethylether gegeben und dazu 7 ml Methyleniodid innerhalb von 10 Minuten zugetropft. Anschließend wird 20 Stunden bei 50°C gerührt. Dann wird das Reaktionsgemisch unter Kühlung mit einem Lösungsmittelgemisch bestehend aus 6 ml Pyridin, 10 ml Diethylether und 6 ml Pentan vorsichtig versetzt und die gesamte Mischung über basisches oder neutrales Aluminiumoxid filtriert und mit Ether/Pentan erschöpfend nachgewaschen. Die vereinigten Filtrate werden zur Trockne eingeengt und der verbleibende Rückstand an basischem Aluminiumoxid flash chromatographiert. Als mobile Phase dient ein Benzol/Pentan-Gemisch (1:1). Nach Umkristallisation aus Hexan bzw. Ethanol werden 0,64 g des Cyclopropanoproduktes erhalten.
Schmelzpunkt (aus Ethanol): 106°C bis 107°C

c) **17β-(Dimethyl-tert.butyl-silyloxy)-3-methoxy-16α,17α-methylen-estra-2,5(10)-dien**

Zu einer frisch bereiteten Birch-Lösung, bestehend aus 40 ml Tetrahydrofuran, 200 ml Ammoniak, 8 ml tert. Butanol und 0,25 g Lithium, werden 5 ml einer 0,42 g 17β-(Dimethyl-tert.butyl-silyloxy)-3-methoxy-16α,17α-methylen-estra-1,3,5(10)-trien enthaltenden Tetrahydrofuranlösung bei ca. -35°C langsam zugetropft. Nach 1,5stündigem Rühren unter Rückfluß wird die noch blaue Lösung durch Zugabe von Ammoniumchlorid (Überschuß) entfärbt, anschließend das Ammoniak abgedampft, die verbleibende Suspension über neutrales Aluminiumoxid filtriert, mit Ether gründlich nachgewaschen und die vereinigten Filtrate im Vakuum zur Trockne eingeengt. Man erhält 0,42 g eines farblosen Öles als dünnschichtchromatographisch einheitliches Produkt, das in dieser Form in die nächste Stufe eingesetzt wird.
IR[$cm^{-1}$]: 1690 und 1655 (Dienolether)

d) **17β-(Dimethyl-tert.butyl-silyloxy)-16α,17α-methylen-estr-5(10)-en-3-on**

0,42 g 17β-(Dimethyl-tert.butyl-silyloxy)-16α,17α-methylen-estra-2,5(10)-dien werden in 12 ml 80prozentigem wäßrigen Aceton suspendiert und mit 0,012 ml 25prozentiger Schwefelsäure versetzt. Man rührt 2 Stunden bei Raumtemperatur und gibt anschließend langsam Eiswasser hinzu, wobei das Steroid ausfällt. Man saugt das Steroid ab, wäscht dieses mit Bicarbonatlösung und zuletzt mit Wasser neutral. Man erhält 0,3 g des amorph kristallinen 3-Keto-5(10)-produktes, das in dieser Form in die nächste Stufe eingesetzt wird.
IR[$cm^{-1}$]: 1707 (C=O)

e) **17β-(Dimethyl-tert.butyl-silyloxy)-16α,17α-methylen-estra-4,9-dien-3-on**

0,35 g 17β-(Dimethyl-tert.butyl-silyloxy)-16α,17α-methylen-estr-5(10)-en-3-on werden in 10 ml Pyridin (KOH getrocknet) gelöst und bei -30°C unter Inertgas mit 0,45 g Pyridinhydrobromidperbromid versetzt. Danach rührt man 20 Minuten bei -5°C, vernichtet anschließend überschüssiges Bromierungsmittel durch Zugabe von 2-Methylbut-2-en. Darin wird 4 Stunden bei Raumtemperatur gerührt. Nach erfolgter Umsetzung wird in Eiswasser eingerührt und das Steroid mit Ether extrahiert. Der nach dem Einengen verbleibende Rückstand wird an basischem Aluminiumoxid chromatographiert mit Benzol/Essigester (10:1). Es werden 0,2 g des 4,9-Dien-3-on-silylethers erhalten, welchen man aus wäßrigem Methanol kristallisieren kann. Schmelzpunkt: 114,5°C bis 115,5°C $[\alpha]_D$: -138,8°

f) **17β-(Dimethyl-tert.butyl-silyloxy)-3,3-ethylendioxy-16α,17α-methylen-estra-5(10),9(11)-dien**

4,3 g 17β-(Dimethyl-tert.butyl-silyloxy)-16α,17α-methylen-estra-4,9-dien-3-on werden in 100 ml Benzol gelöst, mit 5 ml Glykol und 0,2 g p-Toluolsulfonsäure versetzt und 1 Stunde am Wasserabscheider gekocht. Anschließend wird in eine gesättigte Natriumbicarbonatlösung eingerührt und das Steroid mit Benzol extrahiert. Nach dem Einengen der Extrakte wird der verbleibende, dünnschichtchromatographisch einheitliche ölige Rückstand aus wäßrigem Methanol kristallisiert.
Schmelzpunkt: 68,5°C bis 70°C

g) **17β-(Dimethyl-tert.butyl-silyloxy)-3,3-ethylendioxy-5α,10α-epoxy-16α,17α-methylen-estr-9(11)-en**

1 g 17β-(Dimethyl-tert.butyl-silyloxy)-3,3-ethylendioxy-16α,17α-methylen-estra-5(10),9(11)-dien wird in 22,5 ml Acetonitril und 10 ml Methylenchlorid gelöst und unter Eiskühlung mit 0,722 g m-Chlorperbenzoesäure versetzt. Man rührt 15 Minuten unter Beibehaltung der Kühlung, gibt anschließend eine gesättigte, wäßrige Natriumbicarbonatlösung hinzu und extrahiert das Steroid mit Methylenchlorid, nachdem man vorher dem Methylenchlorid 3 Tropfen Triethylamin beimengt. Nach dem Einengen der Extrakte wird an basischem Aluminiumoxid flash chromatographiert. Als mobile Phase dient Benzol. Man erhält 0,8 g 5α,10α-Epoxid in Form eines Öles, welches in dieser Form in die nächste Stufe eingesetzt wird.
IR[$cm^{-1}$]: kein C=O, kein OH

h) **11β-(4-Dimethylaminophenyl)-17β-(dimethyl-tert.butyl-silyloxy)-3,3-ethylendioxy-16α,17α-methylen-estr-9-en-5α-ol**

Zu einer Suspension von 0,48 g Magnesiumspänen in 10 ml Tetrahydrofuran fügt man 0,05 ml 1,2-Dibromethan hinzu und versetzt unter Argon sukzessive mit einer Lösung von 4,2 g p-Brom-dimethylaminobenzol in 30 ml Tetrahydrofuran, wobei die Innentemperatur 50°C nicht übersteigen sollte. Von der so erzeugten p-Dimethylaminophenylmagnesiumbromidlösung entnimmt man 30 ml und versetzt unter Kühlung (-15°C) mit 0,18 g CuCl. Man rührt etwa 15 Minuten unter Beibehaltung dieser Temperatur und fügt dann eine Lösung von 1,8 g 17β-(Dimethyl-tert.butyl-silyloxy)-3,3-ethylendioxy-16α,17α-methylen-5α,10α-epoxy-estr-9(11)-en in 6 ml Tetrahydrofuran tropfenweise hinzu. Anschließend wird 2,5 Stunden bei einer Temperatur von etwa 0°C gerührt, danach wäßrige Ammoniumchloridlösung zugesetzt und das Steroid mit Methylenchlorid extrahiert. Nach dem Einengen der Extrakte wird der Rückstand an basischem Aluminiumoxid (Greiz-Dölau) flash chromatographiert. Als mobile Phase dient Benzol/Essigester (40:1). Man erhält 1,7 g der angereicherten 11β-Dimethylaminoverbindung, die als Öl direkt weiterverarbeitet werden kann.
IR[$cm^{-1}$]: 3500 (OH, assoziiert), 1610 und 1500 (Aromat)

i) **11β-(4-Dimethylaminophenyl)-17β-(dimethyl-tert.butyl-silyloxy)-16α,17α-methylen-estra-4,9-dien-3-on**

0,4 g 11β-(4-Dimethylaminophenyl)-17β-(dimethyl-tert.butyl-silyloxy)-3,3-ethylendioxy-16α,17α-methylen-estr-9-en-5α-ol werden in 10 ml 70prozentiger wäßriger Essigsäure gelöst und 2 Stunden auf dem Wasserbad bei 60°C gerührt. Nach erfolgter Umsetzung fällt man das Steroid durch Zugabe von Wasser und etwas verdünnter Ammoniaklösung aus. Das so erhaltene Rohprodukt wird durch Chromatographie an neutralem, Aluminiumoxid (Greiz-Dölau) mit Benzol/Essigester (20:1) gereinigt. Man erhält 0,31 g der 11β-Dimethylaminoverbindung, die aus Methanol/Wasser kristallisiert werden kann.
Schmelzpunkt: 100°C bis 102,5°C $[\alpha]_D$: 222,3°

Beispiel 16

a) **3-Methoxy-17-trimethylsilyloxy-estra-1,3,5(10),16-tetraen**

20,3 g (72 mmol) 3-Methoxy-estra-1,3,5(10)-trien-17-on werden unter Feuchtigkeitsausschluß in 80 ml Acetonitril ($P_2O_5$ und Molsieb getrocknet) suspendiert und mit 17,4 ml Triethylamin (125 mmol, über KOH getrocknet) sowie mit 12,24 ml Trimethylchlorsilan (96 mmol) bei einer Innentemperatur von 35°C versetzt. Anschließend tropft man zu dieser Suspension eine NaI-Lösung, die 15 g NaI (96 mmol) in 100 ml Acetonitril enthält (NaI bei 140°C im Vakuum getrocknet), so zu, daß die Reaktionstemperatur ohne externe Erwärmung bei 35°C gehalten wird. Die unter Stickstoff durchgeführte Reaktion wird bei starker Rührung vorgenommen. Man erkennt, daß nach erfolgter Zugabe die Menge an nicht gelöstem Ausgangsprodukt zurückgeht. Später sind die Kristalle vollständig verschwunden. Nach 1,5 Stunden beginnt der Silylenolehter auszukristallisieren. Nach 4 Stunden wird die Kristallsuspension in Eiswasser, dem etwas Triethylamin beigefügt wurde, eingerührt. Danach wird abgefrittet, mit Wasser nachgewaschen, trockengesaugt und die Substanz im Vakuum über $P_2O_5$ getrocknet. Man erhält 25,4 g Silylenolether, der aus Hexan oder Methanol umkristallisiert werden kann.
Schmelzpunkt: 80°C bis 84°C

b) **3-Methoxy-16α,17α-methylen-17β-trimethylsilyloxy-estra-1,3,5(10)-trien**

10 g 3-Methoxy-17-trimethylsilyloxy-estra-1,3,5(10), 16-tetraen werden in 20 ml Benzol und 20 ml Dimethoxyethan gelöst und unter Inertgas mit 20 g Zn/Cu-Katalysator (Le Goff) versetzt. Zu diesem Reaktionsgemisch werden unter gelindem Erwärmen und kräftigem Rühren sukzessive 15 ml Methyleniodid so zugegeben, daß eine Reaktionstemperatur von 50°C in etwa eingehalten wird. Anschließend wird bei 50°C 4 Stunden gerührt. Nach erfolgter Umsetzung wird vom Katalysator abfiltriert, das Filtrat mit wäßriger Ammoniumchloridlösung versetzt und das Steroid mit Benzol extrahiert. Der nach dem Einengen der Extrakte verbleibende Rückstand wird an basischem Aluminiumoxid mit einem Benzol/n-Hexan-Gemisch (1:1) flash chromatographiert. Man erhält 4,4 g (53 % d. Th.) der 16α,17α-Methylenverbindung, die aus n-Hexan kristallisiert werden kann. Schmelzpunkt: 118°C bis 119°C

c) **3-Methoxy-16α,17α-methylen-17β-trimethylsilyloxy-estra-2,5(10)-dien.**

Zu einer frisch bereiteten homgenen Birch-Lösung, bestehend aus 250 ml Tetrahydrofuran, 650 ml flüssigem Ammoniak, 30 ml tert.Butanol und 1,9 g Lithium, werden 50 ml einer 10 g 3-Methoxy-16α,17α-methylen-17β-trimethylsilyloxy-estra-1,3,5(10)-trien enthaltenden Tetrahydrofuranlösung bei ca. -35°C langsam zugetropft. Nach 4stündigem Rühren wird die noch blaue Lösung durch Zugabe von Ammoniumchlorid (Überschuß) entfärbt, anschließend das Ammoniak abgedampft, die verbleibende Suspension über neutrales Aluminiumoxid filtriert, mit Ether gründlich nachgewaschen und die vereinigten Filtrate in Vakuum zur Trockne eingeengt.

Man erhält 10,3 g eines farblosen Öles, welches aus n-Hexan kristallisiert werden kann.
Schmelzpunkt: 107,5°C bis 112,5°C

d) **17β-Hydroxy-16α,17α-methylen-estr-5(10)-en-3-on**

3,32g 3-Methoxy-16α,17α-methylen-17β-trimethylsilyloxy-estra-2,5(10)-dien werden in 40 ml 80prozentigem wäßrigen Aceton gelöst und mit 0,16 ml 20prozentiger Schwefelsäure versetzt. Man rührt 2 Stunden bei Raumtemperatur und gibt anschließend langsam Eiswasser hinzu, wobei das Steroid ausfällt. Man saugt das Steroid ab, wäscht dieses mit Bicarbonatlösung und zuletzt mit Wasser neutral. Man erhält 2,3 g der 3-Keto-5(10)-Verbindung, die aus wäßrigem Methanol umkristallisiert werden kann.
Schmelzpunkt: 141°C bis 147°C

e) **17β-Acetoxy-16α,17α-methylen-estra-4,9-dien-3-on**

Ca. 5,2 g des bei der Bromierung/Dehydrobromierung von 17β-Hydroxy-16α,17α-methylen-estr-5(10)-en-3-on analog 15e) erhaltenen Dienons werden ohne Aufarbeitung direkt in der Pyridinlösung acetyliert. Man verfährt dabei so, daß man nach abgeklungener Dehydrobromierung das Reaktionsgemisch mit 6 ml Acetanhydrid versetzt und 16 Stunden bei Raumtemperatur rührt. Nach erfolgter Umsetzung wird in Eiswasser, dem etwas Salzsäure zugefügt wurde, eingerührt und das Steroid mit Methylenchlorid extrahiert. Der nach dem Einengen der Extrakte verbleibende Rückstand wird an neutralem Aluminiumoxid chromatogrphiert. Als mobile Phase dient ein Benzol/Essigester-Gemisch (10:1). Es werden 3,99 g des aus Heptan/Benzol kristallisierenden Produktes erhalten.
Schmelzpunkt: 137°C bis 138,5°C $[\alpha]_D$: 168,1°

f) **17β-Acetoxy-3,3-ethylendioxy-16α,17α-methylen-estra-5(10),9(11)-dien**

5 g 17β-Acetoxy-16α,17α-methylen-estra-4,9-dien-3-on werden in 100 ml Benzol gelöst, mit 5 ml Glygol und 0,2 g p-Toluolsulfonsäure versetzt und 2 Stunden am Wasserabscheider gekocht. Anschließend wird in eine wäßrige gesättigte Natriumbicarbonatlösung eingerührt und das Steroid mit Benzol extrahiert. Nach Chromatographie an basischem Aluminiumoxid und Einengen der Eluate wird der dünnschichtchromatographisch einheitliche Rückstand direkt weiterverarbeitet.
IR: kein C=O

g) **17β-Acetoxy-3,3-ethylendioxy-5α,10α-epoxy-16α,17α-methylen-estr-9(11)-en**

5 g 17β-Acetoxy-3,3-ethylendioxy-16α,17α-methylen-estra-5(10),9(11)-dien, 2 g wasserfreies $Na_2HPO_4$ und 1 g $Na_2CO_3$ werden in 45 ml Methylenchlorid suspendiert und unter Rühren bei Raumtemperatur mit 7,25 ml 30prozentigem $H_2O_2$ und zuletzt mit 1,25 g Chloralhydrat versetzt. Dann wird 20 Stunden bei Raumtemperatur gerührt und im Anschluß daran eine wäßrige Natriumcarbonatlösung zugegeben und das Steroid mit Methylenchlorid extrahiert. Die organische Phase wird noch zweimal mit einer Natriumcarbonatlösung und zuletzt mit Wasser gewaschen, anschließend getrocknet und eingeengt. Der verbleibende Rückstand wird an basischem Aluminiumoxid mit Benzol/Essigester (20:1 bis 9:1) flash chromatographiert. Man erhält 4,25 g 5α,10α-Epoxid als Öl, welches direkt in die nächste Stufe eingesetzt wird.

h) **11β-(4-Acetylphenyl)-16α,17α-methylen-estra-4,9-dien-17β-ol**

Die Herstellung der Stufe h erfolgt analog dem Beispiel 15

i) **17β-Acetoxy-11β-(4-acetylphenyl)-16α,17α-methylen-estra-4,9-dien-3-on**

0,4 g 11β-(4-Acetylphenyl)-16α,17α-methylen-estra-4,9-dien-17β-ol werden in 2 ml Pyridin gelöst und nach Zugabe von 1 ml Essigsäureanhydrid und 0,005 g Dimethylaminopyridin bei Raumtemperatur 16 Stunden stehen gelassen. Anschließend wird in Eiswasser eingerührt und das ausgefallene Produkt abgefrittet. Zur Reinigung wird das Rohprodukt an neutralem Aluminiumoxid mit Benzol/Essigester (10:1) flash chromatographiert. Man erhält 0,25 g 11β-Acetoxy-4,9-dien-3-on, welches aus Methanol umkristallisiert werden kann.
Schmelzpunkt: 108°C bis 111°C $[\alpha]_D$: 257,3°

Beispiel 17

Die Herstellung der Stufen a bis d erfolgt analog zum Beispiel 16

e) **17β-Hydroxy-16α,17α-methylen-estra-4,9-dien-3-on**

2,3 g 17β-Hydroxy-16α,17α-methylen-estr-5(10)-en-3-on werden in 20 ml über KOH getrocknetem Pyridin gelöst und bei -30°C unter Rühren in einer Inertgasatmosphäre mit 2,88 g Pyridinhydrobromidperbromid versetzt. Danach rührt man etwa 15 Minuten bei einer Temperatur von ca. -5°C und vernichtet anschließend das überschüssige Bromierungsmittel durch Zugabe von Dihydropyran. Dann wird 4 Stunden bei Raumtemperatur gerührt, das Reaktionsgemisch mit Wasser versetzt und das Steroid mit Methylenchlorid extrahiert. Nach dem Einengen der Extrakte wird der verbleibende Rückstand an Kieselgel chromatographiert. Als mobile Phase dient ein Benzol/Essigester-Gemisch (5:1 bis 2:1). Man erhält 1,6 g des Dienons, welches aus wäßrigem Methanol kristallisiert werden kann. Schmelzpunkt: 191°C bis 193°C $[\alpha]_D$: -139,1°

f) **17β-(Dimethyl-tert.butyl-silyloxy)-16α,17α-methylen-estra-4,9-dien-3-on**

Das nach der Bromierung/Dehydrobromierung von 17β-Hydroxy-16α,17α-methylen-estr-5(10)-en-3-on analog 15 e) erhaltene Dienon wird ohne Aufarbeitung direkt in der Pyridinlösung silyliert. Man verfährt dabei so, daß man nach abgeklungener Dehydrobromierung 2,41 g (16 mmol) Dimethyl-tert.butyl-chlorsilan und 3,4 g (50 mmol) Imidazol zufügt und 2 bis 2,5 Stunden bei Raumtemperatur rührt. Dann wird mit Eiswasser und verdünnter Salzsäure versetzt und das Steroid mit Ether extrahiert. Der nach dem Einengen erhaltene Rückstand wird an basischem Aluminiumoxid mit Benzol/Essigester (10:1) chromatographiert. Es werden 2,35 g (68 % d. Th.) Silylether erhalten, welcher aus wasserhaltigem Methanol kristallisiert werden kann.
Schmelzpunkt: 114,5°C bis 115,5°C $[\alpha]_D$: -138,8°

h) **17β-(Dimethyl-tert.butyl-silyloxy)-3,3-ethylendioxy-11β-(4-methoxyphenyl)-16α,17α-methylen-estr-9-en-5α-ol**

Von einer durch Umsetzung von 0,48 g Magnesiumspänen und 2,36 ml p-Bromanisol in 20 ml Tetrahydrofuran bei 35°C dargestellten 4-Methoxyphenylmagnesiumbromidlösung werden 20 ml entnommen und unter Argon und Kühlung auf -5° C bis -15° C mit 0,1 g CuCl versetzt. Man rührt 15 Minuten unter Beibehaltung der Kühlung und fügt dann eine Lösung von 1 g 17β-(Dimethyl-tert.butyl-silyloxy)-3,3-ethylendioxy-16α,17α-methylen-5α,10α-epoxy-estr-9(11)-en in 4 ml Tetrahydrofuran tropfenweise hinzu. Anschließend wird 30 Minuten bei Raumtemperatur gerührt, danach eine wäßrige Ammoniumchloridlösung zugesetzt und das Steroid mit Methylenchlorid extrahiert. Nach dem Einengen der Extrakte wird der Rückstand an bas. Aluminiumoxid (Greiz-Dölau) mit Benzol/Essigester (40:1) flash chromatographiert. Es werden 0,85 g der 11β-Anisylverbindung als Öl isoliert.
IR[$cm^{-1}$]: 3500 (OH, assoziiert), 1605, 1580 und 1500 (Aromat)

i) **17β-Hydroxy-16α,17α-methylen-11β-(4-methoxyphenyl)-estra-4,9-dien-3-on**

0,94 g 17β-(Dimethyl-tert.butyl-silyloxy)-3,3-ethylendioxy-11β-(4-methoxyphenyl)-16α,17α-methylen-estr-9-en-5α-ol werden in 10 ml 70prozentiger wäßriger Essigsäure gelöst und 2 Stunden auf dem Wasserbad bei 60°C gerührt. Nach erfolgter Umsetztung fällt man das Steroid durch Zugabe von Wasser aus. Das so erhältliche Rohprodukt wird durch Chromatographie an neutralem, Aluminiumoxid (Greiz-Dölau), als mobile Phase dient ein Benzol/Essigester-Gemisch (3:1), gereinigt. Man erhält 0,23 g der 11β-Anisylverbindung, die aus Methanol/Wasser kristallisiert werden kann.
Schmelzpunkt: 107°C bis 110°C $[\alpha]_D$: 210,1°

Beispiel 18

Die Herstellung der Stufen a bis g erfolgt analog zum Beispiel 15

h) **17β-(Dimethyl-tert.butyl-silyloxy)-3,3-ethylendioxy-16α,17α-mehtylen-11β-[4-(2'-methyl-1',3'-dioxolan-2-yl-)phenyl]-estr-9-en-5α-ol**

Zu einer Suspension von 0,48 g Magnesiumspänen in 13 ml Tetrahydrofuran fügt man 0,05 ml 1,2-Dibromethan hinzu und versetzt unter Argon sukzessive mit einer Lösung von 4,9 g p-Brom-(2-methyl-1',3'-dioxolan-2'-yl-)benzol in 27 ml Tetrahydrofuran, wobei die Innentemperatur 45°C nicht übersteigen sollte. Nach Auflösen des Magnesiums entnimmt man 30 ml 4-(2'-Methyl-1',3'-dioxolan-2'-yl-)phenyl-magnesiumbromidlösung und gibt dazu unter Kühlung (-5°C bis -15°C) 0,2 g CuCl. Man rührt 15 Minuten unter Beibehaltung dieser Temperatur und fügt dann eine Lösung von 1,62 g 17β-(Dimethyl-tert.butyl-silyloxy)-3,3-ethylendioxy-16α,17α-methylen-5α,10α-epoxy-estr-9(11)-en in 7 ml Tetrahydrofuran tropfenweise hinzu. Anschließend wird eine Stunde gerührt, wobei die Reaktionslösung allmählich auf Raumtemperatur gebracht wird. Nach erfolgter Umsetzung wird eine wäßrige Ammoniumchloridlösung zugesetzt und das Steroid mit Methylenchlorid extrahiert. Nach dem Einengen der Extrakte wird der verbleibende Rückstand an basischem Aluminiumoxid (Greiz-Dölau) mit Benzol/Essigester (20:1) flash chromatographiert. Man erhält 1,6 g der Acetophenylketalverbindung, die aus Methanol umkristallisiert werden kann.
Schmelzpunkt: 144°C bis 152°C (semikristallin)

i) **11β-(4-Acetylphenyl)-17β-hydroxy-16α,17α-methylen-estra-4,9-dien-3-on**

0,4 g 17β-(Dimethyl-tert.butyl-silyloxy)-3,3-ethylendioxy-16α,17α-methylen-11β-[4-(2'-methyl-1',3'-dioxolan-2'-yl-)phenyl]-estr-9-en-5α-ol werden in 10 ml 70prozentiger wäßriger Essigsäure gelöst und 2 Stunden auf dem Wasserbad bei 60°C gerührt. Nach erfolgter Umsetzung fällt man das Steroid durch Zugabe von Wasser aus. Das erhaltene Rohprodukt wird durch Chromatographie an neutralem Aluminiumoxid (Greiz-Dölau), als mobile Phase dient ein Benzol/Essigester-Gemisch (5:1), gereinigt. Man erhält 0,28 g der 11β-Acetophenylverbindung, die aus Methanol/Wasser kristallisiert werden kann
Schmelzpunkt: 123°C bis 128°C $[\alpha]_D$: 268,7°

Beispiel 19

Die Herstellung der Stufen a bis e erfolgt analog zum Beispiel 16.

f) **3,3-Etyhlendioxy-17β-methoxymethylenoxy-16α,17α-methylen-estra-5(10),9(11)-dien**

3,6 g 17β-Acetoxy-3,3-ethylendioxy-16α,17α-methylen-estra-5(10),9(11)-dien werden unter Argon in 25 ml absolutem Tetrahydrofuran gelöst und nach Zugabe von 0,512 g Naphthalin und 0,2 g Lithium 3 Stunden bei 50°C gerührt. Man läßt auf Raumtemperatur abkühlen, versetzt anschließend mit einer 1,5 ml Chlormethylmethylether enthaltenden Tetrahydrofuranlösung und läßt über Nacht stehen. Anschließend gibt man Wasser hinzu und extrahiert das Steroid mit Ether. Der nach dem Einengen erhältliche Rückstand wird an basischem Aluminiumoxid chromatographiert. Als mobile Phase dient ein Benzol/Essigester-Gemisch (10:1). Es werden 2,5 g des 17β-Methoxymethylethers als Öl erhalten, das direkt in die nächste Stufe eingesetzt wird.
IR: kein C=O

g) **3,3-Ethylendioxy-17β-methoxymethylenoxy-16α,17α-methylen-5α,10α-epoxy-estr-9(11)-en**

Die Herstellung erfolgt analog Beispiel 15 g).
Die Verbindung wird als Öl isoliert.
IR[$cm^{-1}$]: kein C=O

h) **3,3-Ethylendioxy-17β-methoxymethylenoxy-11β-[4-(2'-methyl-1',3'-dioxolan-2'-yl-)phenyl]-16α,17α-methylen-estr-9-en-5α-ol**

Die Herstellung erfolgt analog dem Beispiel 18 h).
Schmelzpunkt (aus Methanol): 150°C bis 155°C $[\alpha]_D$: 44,9°

i) **11β-(4-Acetylpheyl)-17β-methoxymethylenoxy-16α,17α-methylen-estra-4,9-dien-3-on**

Die Herstellung erfolgt analog Beispiel 17 i).
Schmelzpunkt: 70°C bis 74°C $[\alpha]_D$: 290,1°

Beispiel 20

Die Herstellung der Stufen a bis h erfolgt analog zum Beispiel 15.

i) **11β-(4-Dimethylaminophenyl)-17β-hydroxy-16α,17α-methylen-estra-4,9-dien-3-on**

0,5 g 11β-(4-Dimethylaminophenyl)-17β-(dimethyl-tert.butyl-silyoxy)-3,3-ethylendioxy-16α,17α-methylen-estr-9-en-5α-ol werden in 10 ml 70prozentiger wäßriger Essigsäure gelöst und 10 Stunden auf dem Wasserbad bei einer Badtemperatur von 70°C gerührt. Nach erfolgter Umsetzung wird das Steroid durch Zugabe von Wasser und etwas verdünnter Ammoniaklösung ausgefällt. Das Rohprodukt wird durch Chromatographie an neutralem Aluminiumoxid (Greiz/Dölau) mit Benzol/Essigester (5:1) gereinigt. Man erhält 0,3 g der 11β-Dimethylaminophenylverbindung, die aus Methanol/Wasser kristallisiert werden kann.
Schmelzpunkt: 137°C bis 140°C $[\alpha]_D$: 287,6°

Beispiel 21

Herstellung von 17-Alkoxy-16α,17α-methylenverbindungen

a) **3-Methoxy-17-trimethylsilyloxy-estra-1,3,5(10),16-tetraen**

10 g 3-Methoxy-estra-1,3,5(10)-trien-17-on (35,2 mmol) werden unter Feuchtigkeitsausschluß unter Inertgas mit 30 ml über $P_2O_5$ destilliertem Methylenchlorid und 10,5 g Trifluormethansulfonsäuretrimethylsilylester (47,2 mmol, D = 1,15) versetzt. Anschließend gibt man portionsweise unter Rühren und gelinder Kühlung ca. 7 ml Triethylamin (KOH getrocknet, M = 101,2; D = 0,726) hinzu, wobei die Lösung deutlich alkalisch reagiert. Dann wird 12 Stunden bei Raumtemperatur stehen gelassen und nach erfolgter Umsetzung das Lösungsmittel im Vakuum abgezogen. Der verbleibende dickflüssige Rückstand wird dreimal mit ca. 20 ml absolutem Ether unter Inertgas extrahiert. Die etherische Lösung, in der sich das Steroid befindet, wird direkt in die nächste Stufe eingesetzt.

Die Herstellung der Stufe b erfolgt analog dem Beispiel 16.

b') **17β-Hydroxy-3-methoxy-16α,17α-methylen-estra-1,3,5(10)-trien**

0,2 g 3-Methoxy-16α,17α-methylen-17β-trimethylsilyloxy-estra-1,3,5(10)-trien werden zusammen mit 5 ml Methanol und 0,05 g Pyridiniumtosylat 30 Minuten auf dem Wasserbad erwärmt. Nach erfolgter Verseifung wird mit Natriumbicarbonatlösung versetzt und das Steroid mit Methylenchlorid extrahiert. Nach dem Einengen der Extrakte wird aus Methanol/Wasser umkristallisiert, wobei 0,13 g der 17β-Hydroxyverbindung erhalten werden.
Schmelzpunkt: 114°C bis 116°C

b") **3,17-Dimethyoxy-16α,17α-methylen-estra-1,3,5(10)-trien**

0,1 g 17β-Hydroxy-3-methoxy-16α,17α-methylen-estra-1,3,5(10)-trien werden in 5 ml absolutem Tetrahydrofuran gelöst und bei Raumtemperatur mit einer 0,5 molaren Lithiumnaphthalidlösung in Tetrahydrofuran tropfenweise versetzt, bis die Lösung nicht mehr entfärbt wird. Im Anschluß daran gibt man 0,1 ml Methyliodid zu und rührt unter gelindem Erwärmen etwa 30 Minuten. Nach erfolgter Umsetzung gibt man Wasser hinzu und extrahiert das Steroid mit Ether. Der nach dem Einengen der Etherextrakte verbleibende Rückstand wird an neutralem Aluminiumoxid chromatographiert. Die Elution erfolgt mit Benzol. Man erhält 0,1 g des 17β-Methylethers, der aus Methanol kristallisiert werden kann. Schmelzpunkt: 128°C bis 131,5°C $[\alpha]_D$: 93,5°

## Patentansprüche

1. 11β-substituierte 16α,17α-Methylen-estra-4,9-dien-3-one der allgemeinen Formel I

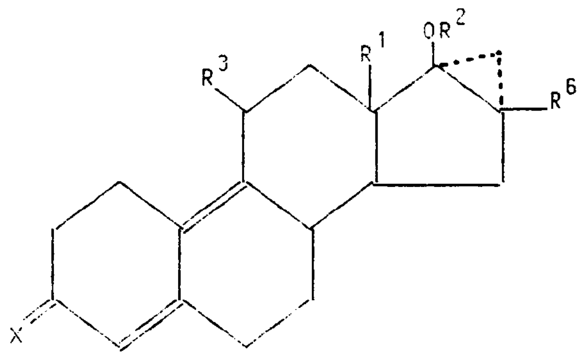

(I)

worin

$R^1$ eine Methyl- oder Ethylgruppe,

$R^2$ ein Wasserstoff, eine Alkyl-, Alkoxymethyl-, Alkanoyl-, Alkoxycarbonylgruppe - jeweils mit einer Kohlenstoffkette von 1 bis 6 Kohlenstoffatomen, 2-Methoxyethyl-, 2-Hydroxyethyl-, 2-Alkanoyloxyethyl-, (Alkanoyl = $C_1$-$C_4$) oder eine Trialkylsilylgruppe mit Alkylresten von 1 bis 4 Kohlenstoffatomen,

$R^3$ eine Vinyl-, $C_1$- bis $C_6$-Alkyl-Gruppe oder einen para-substituierten Phenylrest mit $-OCH_3$, $-SCH_3$, $-NH(CH_3)_2$, $-NHCH_3$, -CN, -CHO, $CH_3CO$, $CH_3CHOH$ oder $CH_2OH$ als para-Substituent,

$R^6$ ein Wasserstoffatom oder eine Alkylgruppe mit 1 bis 4 Kohlenstoffatome sowie

X ein Sauerstoffatom, eine Hydroxy- oder Methoxyiminogruppierung (N-OH bzw. $N-OCH_3$) oder ein cyclisches Thioketal mit 2 oder 3 Kohlenstoffringatomen bedeutet.

**2.** Verbindungen der allgemeinen Formel I, nach Anspruch 1, worin

$R^1$ eine Methylgruppe,

$R^2$ ein Wasserstoffatom, eine $C_1$- bis $C_6$-Alkylgruppe, eine Trialkylsilylgruppe mit Alkylresten mit 1 bis 4 Kohlenstoffatomen, eine Acetyl-, $-CH_2OCH_3$- oder 2-Methoxyethylgruppe,

$R^3$ ein Vinyl- oder para-substituierter Phenylrest mit $-N(CH_3)_2$, -CHO $-C(O)CH_3$, $-OCH_3$ als para-Substituent,

$R^6$ ein Wasserstoffatom oder eine Methylgruppe sowie

X ein Sauerstoffatom bedeutet.

**3.** Verbindungen nach Anspruch 1, nämlich

- 17β-Ethoxy-11β-(4-methoxyphenyl)-16α,17α-methylen-estra-4,9-dien-3-on,
- 11β-(4-Dimethylaminophenyl)-17β-methoxy-16α,17α-methylen-estra-4,9-dien-3-on,
- 11β-(4-Dimethylaminophenyl)-17β-ethoxy-16α,17α-methylen-estra-4,9-dien-3-on,
- 17β-Butyloxy-11β-(4-dimethylaminopohenyl)-16α,17α-methylen-estra-4,9-dien-3-on
- 11β-(4-Acetylphenyl)-17β-methoxy-16α,17α-methylen-estra-4,9-dien-3-on,
- 11β-(4-Acetylphenyl)-17β-ethoxy-16α,17α-methylen-estra-4,9-dien-3-on,
- 11β-(4-Acetylphenyl)-17β-butyloxy-16α,17α-methylen-estra-4,9-dien-3-on,
- 11β-(4-Acetylphenyl)-17β-hexyloxy-16α,17α-methylen-estra-4,9-dien-3-on,
- 11β-(4-Acetylphenyl)-17β-methoxyethyloxy-16α,17α-methylen-estra-4,9-dien-3-on,
- 11β-(4-Formylphenyl)-17β-methoxy-16α,17α-methylen-estra-4,9-dien-3-on sowie
- 17β-Ethoxy-11β-(4-formylphenyl)-16α,17α-methylen-estra-4,9-dien-3-on,
- 17β-(Dimethyl-tert.butyl-siloxy)-11β-(4-methoxyphenyl)-16α,17α-methylen-estra-4,9-dien-3-on,
- 11β-(4-Dimethylaminophenyl)-17β-(dimethyl-tert.butyl-siloxy)-16α,17α-methylen-estra-4,9-dien-3-on,
- 11β-(4-Acetylphenyl)-17β-(dimethyl-tert.butyl-siloxy)-16α,17α-methylen-estra-4,9-dien-3-on,
- 17β-Hydroxy-11β-(4-methoxyphenyl)-16α,17α-methylen-estra-4,9-dien-3-on,
- 11β-(4-Dimethylaminophenyl)-17β-hydroxy-16α,17α-methylen-estra-4,9-dien-3-on,
- 11β-(4-Acetylphenyl)-17β-hydroxy-16α,17α-methylen-estra-4,9-dien-3-on,
- 17β-Acetoxy-11β-(4-dimethylaminophenyl)-16α,17α-methylen-estra-4,9-dien-3-on,
- 17β-Acetoxy-11β-(4-acetylphenyl)-16α,17α-methylen-estra-4,9-dien-3-on,
- 11β-(4-Dimethylaminophenyl)-17β-methoxymethyl-16α,17α-methylen-estra-4,9-dien-3-on,
- 11β-(4-Acetylphenyl)-17β-methoxymethyl-16α,17α-methylen-estra-4,9-dien-3-on,
- 11β-(4-Dimethylaminophenyl)-17β-methoxy-16β-methyl-16α,17α-methylen-estra-4,9-dien-3-on sowie

- 11β-(4-Acetylphenyl)-17β-methoxy-16α,17α-methylen-estra-4,9-dien-3-on.

4. Verfahren zur Herstellung von 11β-substituierten 16α,17α-Methylen-estra-4,9-dien-3-onen der allgemeinen formel I, dadurch gekennzeichnet, daß eine Verbindung der allgemeinen Formel XI

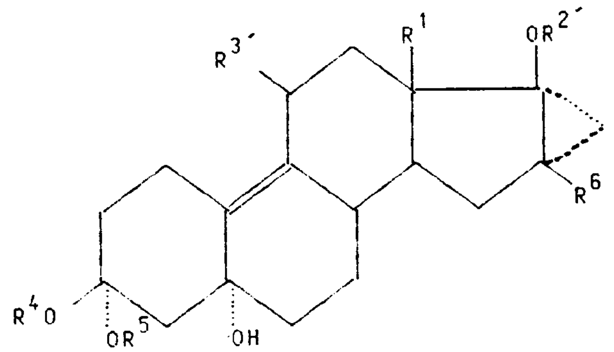

(XI),

worin

| | |
|---|---|
| $R^1$ | ein Wasserstoffatom oder eine Methylgruppe und $R^4$ und $R^5$ je eine Methyl- oder Ethylgruppe oder gemeinsam eine Ethylen- oder 2,2-Dialkylpropylengruppe, insbesondere 2,2-Dimethylpropylengruppe, bedeuten, sowie |
| $R^{2'}$ und $R^{3'}$ | dieselbe Bedeutung wie $R^2$ und X in Formel I haben, wobei gegebenenfalls vorhandene Ketogruppen geschützt sind, |

letztere durch Säurebehandlung in einem mit Wasser mischbaren Lösungsmittel in 11β-substituierte 16α,17α-Methylen-estra-4,9-dien-3-one der allgemeinen Formel I überführt sowie diese anschließend gegebenenfalls durch Oximierung, Thioketalisierung oder Acylierung zu einer anderen Verbindung der allgemeinen Formel I derivatisiert wird.

5. Pharmazeutische Präparate, dadurch gekennzeichnet, daß sie mindestens eine Verbindung der allgemeinen Formel I sowie einen pharmazeutischen verträglichen Träger enthalten.

6. Verwendung von Verbindungen der allgemeinen Formel I zur Herstellung von Arzneimitteln.

## Claims

1. 11β-substituted 16α,17α-methylene-oestra-4,9-dien-3-ones of the general formula I

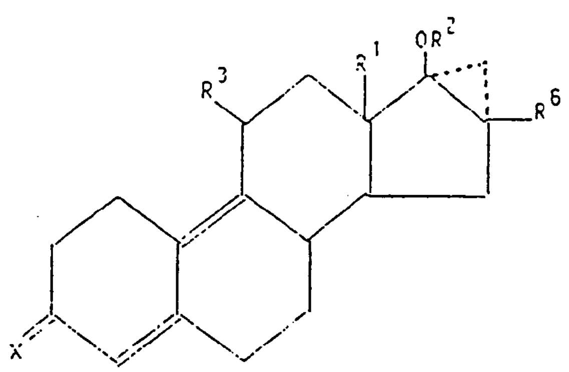

(I)

wherein

| | |
|---|---|
| $R^1$ | represents a methyl or ethyl group, |
| $R^2$ | represents a hydrogen atom, an alkyl, alkoxymethyl, alkanoyl or alkoxycarbonyl group having in each case a carbon chain of from 1 to 6 carbon atoms, 2-methoxyethyl, 2-hydroxyethyl, 2-alkanoyloxyethyl, (alkanoyl-$C_1$-$C_4$) or a trialkylsilyl group having alkyl radicals with from 1 to 4 carbon |

atoms,

$R^3$ represents a vinyl group, a $C_1$- to $C_6$-alkyl group or a para-substituted phenyl radical containing as the para-substituent $-OCH_3$, $-SCH_3$, $-N(CH_3)_2$, $-NHCH_3$, -CN, -CHO, $CH_3CO$, $CH_3CHOH$ or $CH_2OH$,

$R^6$ represents a hydrogen atom or an alkyl group having from 1 to 4 carbon atoms, and

X represents an oxygen atom, a hydroxy- or methoxy-imino grouping (N-OH and $N-OCH_3$, respectively) or a cyclic thioketal having 2 or 3 ring carbon atoms.

2. Compounds of the general formula I according to claim 1, wherein

$R^1$ represents a methyl group,

$R^2$ represents a hydrogen atom, a $C_1$- to $C_6$-alkyl group, a trialkylsilyl group having alkyl radicals with from 1 to 4 carbon atoms, or an acetyl, $-CH_2OCH_3$ or 2-methoxyethyl group,

$R^3$ represents a vinyl group or a para-substituted phenyl radical containing as the para-substituent $-N(CH_3)_2$, -CHO, $-C(O)CH_3$ or $-OCH_3$,

$R^6$ represents a hydrogen atom or a methyl group, and

X represents an oxygen atom.

3. Compounds according to claim 1, namely
17β-ethoxy-11β-(4-methoxyphenyl)-16α,17α-methylene-oestra-4,9-dien-3-one,
11β-(4-dimethylaminophenyl)-17β-methoxy-16α,17α-methylene-oestra-4,9-dien-3-one,
11β-(4-dimethylaminophenyl)-17β-ethoxy-16α,17α-methylene-oestra-4,9-dien-3-one,
17β-butoxy-11β-(4-dimethylaminophenyl)-16α,17α-methylene-oestra-4,9-dien-3-one,
11β-(4-acetylphenyl)-17β-methoxy-16α,17α-methylene-oestra-4,9-dien-3-one,
11β-(4-acetylphenyl)-17β-ethoxy-16α,17α-methylene-oestra-4,9-dien-3-one,
11β-(4-acetylphenyl)-17β-butoxy-16α,17α-methylene-oestra-4,9-dien-3-one,
11β-(4-acetylphenyl)-17β-hexyloxy-16α,17α-methylene-oestra-4,9-dien-3-one,
11β-(4-acetylphenyl)-17β-methoxyethoxy-16α,17α-methylene-oestra-4,9-dien-3-one,
11β-(4-formylphenyl)-17β-methoxy-16α,17α-methylene-oestra-4,9-dien-3-one and
17β-ethoxy-11β-(4-formylphenyl)-16α,17α-methylene-oestra-4,9-dien-3-one,
17β-(dimethyl-tert.-butyl-siloxy)-11β-(4-methoxyphenyl)-16α,17α-methylene-oestra-4,9-dien-3-one,
11β-(4-dimethylaminophenyl)-17β-(dimethyl-tert.-butylsiloxy)-16α,17α-methylene-oestra-4,9-dien-3-one,
11β-(4-acetylphenyl)-17β-(dimethyl-tert.-butyl-siloxy)-16α,17α-methylene-oestra-4,9-dien-3-one,
17β-hydroxy-11β-(4-methoxyphenyl)-16α,17α-methylene-oestra-4,9-dien-3-one,
11β-(4-dimethylaminophenyl)-17β-hydroxy-16α,17α-methylene-oestra-4,9-dien-3-one,
11β-(4-acetylphenyl)-17β-hydroxy-16α,17α-methylene-oestra-4,9-dien-3-one,
17β-acetoxy-11β-(4-dimethylaminophenyl)-16α,17α-methylene-oestra-4,9-dien-3-one,
17β-acetoxy-11β-(4-acetylphenyl)-16α,17α-methylene-oestra-4,9-dien-3-one,
11β-(4-dimethylaminophenyl)-17β-methoxymethyl-16α,17α-methylene-oestra-4,9-dien-3-one,
11β-(4-acetylphenyl)-17β-methoxymethyl-16α,17α-methylene-oestra-4,9-dien-3-one,
11β-(4-dimethylaminophenyl)-17β-methoxy-16β-methyl16α,17α-methylene-oestra-4,9-dien-3-one and
11β-(4-acetylphenyl)-17β-methoxy-16α,17α-methylene-oestra-4,9-dien-3-one.

4. Process for the manufacture of 11β-substituted 16α,17α-methylene-oestra-4,9-dien-3-ones of the general formula I, characterised in that a compound of the general formula XI

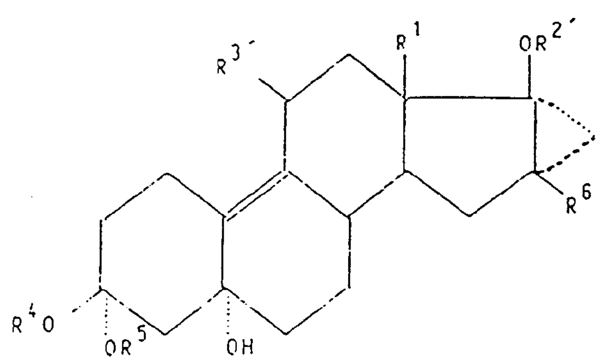

(XI),

wherein

$R^1$ represents a hydrogen atom or a methyl group, and $R^4$ and $R^5$ each represents a methyl or ethyl group or together represent an ethylene or a 2,2-dialkyl-propylene group, especially a 2,2-dimethylpropylene group, and $R^{2'}$ and $R^{3'}$ have the same meanings as $R^2$ and X in formula I, and wherein any keto groups present are protected,

is converted by acid treatment in a water-miscible solvent into an 11β-substituted 16α,17α-methylene-oestra-4,9-dien-3-one of the general formula I, and the latter is then optionally derivatised by oximation, thioketalisation or acylation to form another compound of the general formula I.

**5.** Pharmaceutical preparations, characterised in that they contain at least one compound of the general formula I and a pharmaceutically acceptable carrier.

**6.** The use of compounds of the general formula I for the manufacture of medicaments.

## Revendications

**1.** 16α, 17α-Méthylène-estra-4,9-dién-3-ones 11β-substituées de formule générale I

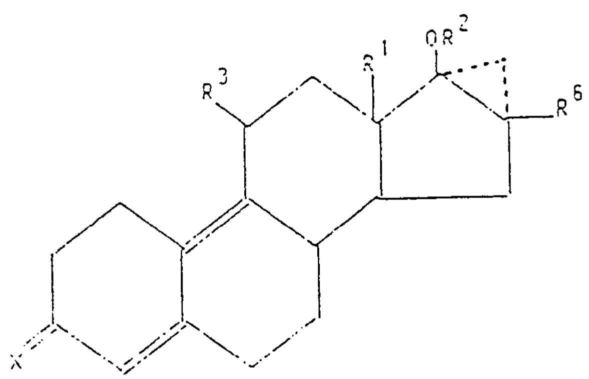

( I )

dans laquelle

| | |
|---|---|
| $R^1$ | représente le groupe méthyle ou éthyle, |
| $R^2$ | un atome d'hydrogène, un alkyle, un alcoxyméthyle, un alcanoyle ou un alcoxycarbonyle avec chacun une chaîne carbonée de 1 à 6 atomes de carbone, ou bien un groupe 2-méthoxyéthyle, 2-hydroxyéthyle, 2-alcanoyloxyéthyle à alcanoyle en $C_1$-$C_4$ ou trialkylsilyle à alkyles en $C_1$-$C_4$, |
| $R^3$ | un groupe vinyle, un alkyle en $C_1$-$C_6$ ou un phényle avec comme substituant à la position para un groupe $-OCH_3$, $-SCH_3$,$-N(CH_3)_2$, $-NHCH_3$, -CN, -CH0, $-CH_3CO$, $-CH_3CHOH$ ou $-CH_2OH$, |
| $R^6$ | un atome d'hydrogène ou un alkyle en $C_1$ à $C_4$, et |
| X | un atome d'oxygène, un groupe hydroxy- ou méthoxyimino (N-OH ou $N-OCH_3$) ou encore un groupe de thiocétal cyclique à 2 ou 3 atomes de carbone de cycle. |

**2.** Composés de formule I selon la revendication 1, dans lesquels

| | |
|---|---|
| $R^1$ | est le groupe méthyle |
| $R^2$ | un atome d'hydrogène, un alkyle en $C_1$ à $C_6$, un groupe trialkylsilyle à alkyles en $C_1$ à $C_4$ ou un groupe acétyle, $-CH_2OCH_3$ ou 2-méthoxyéthyle, |
| $R^3$ | un groupe vinyle ou un phényle avec comme substituant à la position para un groupe $-N(CH_3)_2$, -CHO, $-C(O)CH_3$ ou $-OCH_3$, |
| $R^6$ | un atome d'hydrogène ou le groupe méthyle et |
| X | un atome d'oxygène. |

**3.** Composés selon la revendication 1, à savoir les suivants :

- 17β-Ethoxy-11β-(4-méthoxyphényl)-16α, 17α-méthylène-estra-4,9-dién-3-one,
- 11β-(4-Diméthylaminophényl)-17β-méthoxy-16α, 17α-méthylène-estra-4,9-dién-3-one,
- 11β-(4-Diméthylaminophényl)-17β-éthoxy-16α, 17α-méthylène-estra-4,9-dién-3-one,
- 17β-Butyloxy-11β-(4-diméthylaminopohényl)-16α, 17α-méthylène-estra-4,9-dién-3-one,

- 11β-(4-Acétylphényl)-17β-méthoxy-16α, 17α-méthylène-estra-4,9-dién-3-one,
- 11β-(4-Acétylphényl)-17β-éthoxy-16α, 17α-méthylène-estra-4,9-dién-3-one,
- 11β-(4-Acétylphényl)-17β-butyloxy-16α, 17α-méthylène-estra-4,9-dién-3-one,
- 11β-(4-Acétylphényl-17β-héxyloxy-16α, 17α-méthylène-estra-4,9-dién-3-one,
- 11β-(4-Acétylphényl)-17β-méthoxyéthyloxy-16α, 17α-méthylène-estra-4,9-dién-3-one,
- 11β-(4-Formylphényl)-17β-méthoxy-16α, 17α-méthylène-estra-4,9-dién-3-one,
- 17β-Ethoxy-11β-(4-formylphényl)-16α, 17α-méthylène-estra-4,9-dién-3-one,
- 17β-(Diméthyl-tert.butyl-siloxy)-11β-(4-méthoxyphényl)-16α, 17α-méthylène-estra-4,9-dién-3-one,
- 11β-(4-Diméthylaminophényl)-17β-(diméthyl-tert.butyl-siloxy)-16α, 17α-méthylène-estra-4,9-dién-3-one,
- 11β-(4-Acétylphényl)-17β-(diméthyl-tert.butyl-siloxy)-16α, 17α-méthylène-estra-4,9-diène-3-one,
- 17β-Hydroxy-11β-(4-méthoxyphényl)-16α, 17α-méthylène-estra-4,9-dién-3-one,
- 11β-(4-Diméthylaminophényl)-17β-hydroxy-16α, 17α-méthylène-estra-4,9-dién-3-one,
- 11β-(4-Acétylphényl)-17β-hydroxy-16α, 17α-méthylène-estra-4,9-dién-3-one,
- 11β-(4-Diméthylaminophényl)-17β-méthoxyméthyl-16α, 17α-méthylène-estra-4,9-dién-3-one,
- 17β-Acétoxy-11β-(4-diméthylaminophényl)-16α, 17α-méthylène-estra-4,9-dién-3-one,
- 17β-Acétoxy-11β-(4-acétylphényl)-16α, 17α-méthylène-estra-4,9-dién-3-one,
- 11β-(4-Acétylphényl)-17β-méthoxyméthyl-16α, 17α-méthylène-estra-4,9-dién-3-one,
- 11β-(4-Diméthylaminophényl)-17β-méthoxy-16β-16α, 17α-méthylène-estra-4,9-dién-3-one et
- 11β-(4-Acétylphényl)-17β-méthoxy-16α, 17α-méthylène-estra-4,9-dién-3-one.

4. Procédé de préparation des composés de formule I selon la revendication 1, procédé caractérisé en ce que l'on transforme un composé de formule générale XI

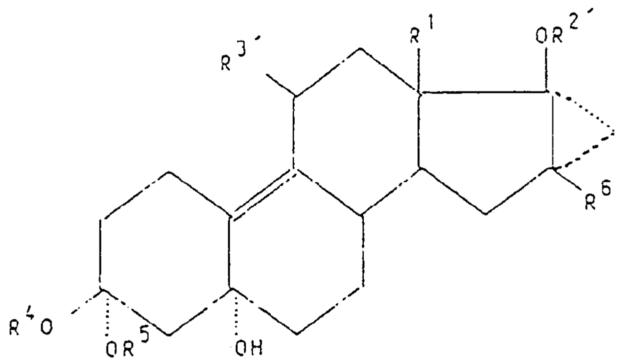

(XI),

(dans laquelle

| | |
|---|---|
| $R^1$ | représente un atome d'hydrogène ou le groupe méthyle et |
| $R^4$ et $R^5$ | sont chacun un groupe méthyle ou éthyle ou bien forment ensemble un groupe éthylène ou 2,2-dialkylpropylène, en particulier 2,2-diméthylpropylène, et $R^{2'}$ et $R^{3'}$ ont les mêmes significations que $R^2$ et X dans la formule I, des groupes céto éventuellement présents étant protégés), |

par traitement avec un acide dans un solvant miscible à l'eau, en une 16α, 17α-méthylène-estra-4,9-dién-3-one 11β-substituée de formule I, que le cas échéant on transforme ensuite en un autre composé de formule I par oximation, thiocétalisation ou acylation.

5. Préparations pharmaceutiques caractérisées en ce qu'elles contiennent un ou plusieurs composés de formule I selon la revendication avec des véhicules ou excipients pharmaceutiques compatibles.

6. L'emploi des composés de formule I selon la revendication 1 pour la préparation de médicaments.